(19) Europäisches Patentamt
European Patent Office
Office européen des brevets

(11) **EP 3 473 219 B1**

(12) **EUROPEAN PATENT SPECIFICATION**

(45) Date of publication and mention
of the grant of the patent:
**25.03.2020 Bulletin 2020/13**

(21) Application number: **16905482.2**

(22) Date of filing: **16.06.2016**

(51) Int Cl.:
***A61F 13/15*** *(2006.01)*   ***B65H 21/02*** *(2006.01)*
***B65H 23/025*** *(2006.01)*   ***B65H 23/188*** *(2006.01)*
***B65H 39/14*** *(2006.01)*

(86) International application number:
**PCT/JP2016/067960**

(87) International publication number:
**WO 2017/216927 (21.12.2017 Gazette 2017/51)**

(54) **METHOD AND DEVICE FOR MANUFACTURING SHEET MEMBER FOR ABSORBENT ARTICLE**

VERFAHREN UND VORRICHTUNG ZUR HERSTELLUNG EINES BLATTELEMENTS FÜR EINEN SAUGFÄHIGEN ARTIKEL

PROCÉDÉ ET DISPOSITIF DE FABRICATION D'UN ÉLÉMENT DE TYPE FEUILLE POUR ARTICLE ABSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**24.04.2019 Bulletin 2019/17**

(73) Proprietor: **Unicharm Corporation**
**Shikokuchuo-shi, Ehime 799-0111 (JP)**

(72) Inventor: **HAGITA, Hiromi**
**Kanonji-shi**
**Kagawa 769-1602 (JP)**

(74) Representative: **Staeger & Sperling**
**Partnerschaftsgesellschaft mbB**
**Sonnenstraße 19**
**80331 München (DE)**

(56) References cited:
| **EP-A1- 1 983 395** | **JP-A- 2002 512 566** |
| **JP-A- 2003 521 339** | **JP-A- 2008 143 699** |
| **JP-A- 2008 143 699** | **JP-B2- 4 246 020** |
| **JP-U- S63 136 660** | **US-A- 4 955 265** |
| **US-A1- 2004 074 942** | **US-A1- 2005 056 678** |

**Description**

[Technical Field]

The present disclosure relates to a method and an apparatus for manufacturing a sheet-like member associated with an absorbent article such as a disposable diaper or the like.

[Background Art]

On a manufacturing line of a disposable diaper as an example of an absorbent article, the continuous sheet is transported in the direction of transport that is a direction in which the continuous sheet continues, so that the continuous sheet is transferred to be supplied to a processing line as an example of a processing section. On the processing line, appropriate processing devices process portions of the continuous sheet, each of which corresponding to a diaper, at intervals in the direction of transport, thereby producing a diaper. When a direction intersecting the direction of transport is defined as a CD direction, a position adjustment device adjusts the positions of end portions in the CD-direction of the continuous sheet that is being transported, so as to be respectively located at target positions in the CD direction, on the processing line.

[Citation List]

[Patent Literature]

[PTL 1] Japanese Patent Application Publication No. 2004-262556
Further relevant prior art is JP2008-143699 and EP1983395 A1.

[Summary of Invention]

[Technical Problem]

In such a position adjustment, a force in CD direction is applied from the position adjustment device to the continuous sheet, and this force may contract or stretch the continuous sheet also in the direction of transport. For example, in the case where the positions of the abovementioned end portions are adjusted to increase a width dimension (dimension in the CD-direction) of the continuous sheet, forces directed outward in the CD direction are applied to both the end portions of the continuous sheet. In this case, the continuous sheet may contract in the direction of transport by a length corresponding to the amount of increase in the width dimension.
Due to such contraction (or stretch) in the direction of transport, a pitch in the direction of transport of the abovementioned processing positions results in a shorter pitch (or a longer pitch) than an appropriate pitch such as a product pitch or the like. Consequently, the abovementioned processing positions of the continuous sheet may deviate from appropriate d positions in the direction of transport.
The present disclosure has been made in view of the above issues, and an object thereof is to reduce the amount of deviation in a direction of transport of a processing position in a continuous sheet from an appropriate position, which may be caused by adjusting, in CD direction, positions of end portions in the CD-direction of the continuous sheet.

[Solution to Problem]

A primary aspect of the present disclosure for achieving an object described above is a method for manufacturing a sheet-like member associated with an absorbent article by using a continuous sheet, the method comprising: a supplying step of transferring and supplying the continuous sheet to a processing section by transporting the continuous sheet in a direction of transport that conforms to a continuous direction in which the continuous sheet continues; a processing step of performing processing onto portions of the continuous sheet at intervals in the direction of transport, at a processing position in the direction of transport, using a processing device in the processing section, the portions each corresponding to the absorbent article; a position adjusting step of adjusting, in a CD direction, a position of an end portion in the CD direction in the continuous sheet, the CD direction intersecting the direction of transport in the continuous sheet, the position adjusting step being performed, by a position adjustment device, at an adjustment processing position in the direction of transport; and a detecting step of detecting a trace of the processing left on the continuous sheet, at a detection processing position downstream in the direction of transport from the processing position and the adjustment processing position, and outputting a detection result, the supplying step including adjusting, based on the detection result, a tension in the direction of transport of the continuous sheet at a time of being transferred to the processing section, at a position upstream in the direction of transport from the processing position; wherein the adjustment processing position is located downstream in the direction of transport from the processing position.
Further, an apparatus for manufacturing a sheet-like member associated with an absorbent article by using a continuous sheet, the apparatus comprising: a supplying section that transfers and supplies the continuous sheet to a processing section by transporting the continuous sheet in a direction of transport that conforms to a continuous direction in which the continuous sheet continues; a processing device that performs processing onto portions of the continuous sheet at intervals in the direction of transport, at a processing position in the direction of transport in the processing section, the portions each corresponding to the absorbent article; a position adjustment device that adjusts, in a CD direction, a position of an end portion in the CD direction of the continuous sheet, at an adjustment processing position in the direction of transport, the CD direction intersecting the direction of transport in the continuous sheet; and a detecting device that detects a trace of the processing left on the continuous sheet, at a detection processing position downstream in the direction of transport from the processing position and the adjustment processing position, and outputs a detection result, the supplying section being configured to adjust, based on the detection result, a tension in the direction of transport of the continuous sheet at a time of being transferred to the processing section, at a position upstream in the direction of transport from the processing position; wherein the adjustment processing position is located downstream in the direction of transport from the processing position.

According to the present disclosure, it is possible to reduce the amount of deviation in a direction of transport of a processing position in a continuous sheet from an appropriate position, which may be caused by adjusting, in CD direction, positions of end portions in the CD-direction of the continuous sheet.

[Brief Description of Drawings]

[FIG. 1] FIG. 1A is a schematic plan view of an example of a disposable diaper 1 in an unfolded state, and FIG. 1B is a cross-sectional view taken along line B-B in FIG. 1A.
[FIG. 2] FIG. 2 is a schematic plan view illustrating a process of production of a diaper 1.
[FIG. 3] FIG. 3 is a schematic side view of a manufacturing line LM of a diaper 1 according to a first embodiment.
[Fig. 4] Fig. 4 is a schematic side view of a top-sheet processing system L3a.
[Fig. 5] Fig. 5A is an enlarged schematic plan view illustrating a CD-direction position adjustment device 35, and Fig. 5B is a cross-sectional view taken along line B-B in Fig. 5A.
[Fig. 6] Fig. 6 is a schematic side view of the top-sheet processing system L3a in the case where an imaging position P36 is located downstream in a direction of transport from a gathering position Pj.
[Fig. 7] Fig. 7 is a schematic side view of a back-sheet processing system L4a.
[Fig. 8] Fig. 8A is an enlarged schematic plan view illustrating a CD-direction position adjustment device 49 provided in the back-sheet processing system L4a, and Fig. 8B is a cross-sectional view taken along line B-B in Fig. 8A.
[Fig. 9] Fig. 9A is a schematic plan view of the top-sheet processing system L3a of a first embodiment, and Fig. 9B is a schematic plan view of the top-sheet processing system L3a of a modified example.

[FIG. 10A] FIG. 10A is a schematic plan view illustrating an example in which two tension adjustment devices 33A and 33B are provided.
[FIG. 10B] FIG. 10B is a schematic side view in which a view along arrows C-C in FIG. 9A and a view along arrows D-D in FIG. 10A are connected and illustrated in a plane.
[Fig. 11] Fig. 11 is a schematic side view of a top-sheet processing line L3aK' in a top-sheet processing system L3a' of a second embodiment.
[Fig. 12] Fig. 12 is a schematic side view of a top-sheet processing line L3aK'' in a top-sheet processing system L3a'' of a third embodiment.
[Fig. 13] Fig. 13 is a schematic side view of a tension adjustment device 133 which is not a dancer-roll type.
[Fig. 14] Fig. 14A is a schematic side view of a CD-direction position adjustment device 135 of another example, and Fig. 14B is a cross-sectional view taken along line B-B in Fig. 14A.

[Description of Embodiments]

At least following matter will become clear from the descriptions of the present specification with reference to the accompanying drawings.
A method for manufacturing a sheet-like member associated with an absorbent article by using a continuous sheet, the method comprising: a supplying step of transferring and supplying the continuous sheet to a processing section by transporting the continuous sheet in a direction of transport that conforms to a continuous direction in which the continuous sheet continues; a processing step of performing processing onto portions of the continuous sheet at intervals in the direction of transport, at a processing position in the direction of transport, using a processing device in the processing section, the portions each corresponding to the absorbent article; a position adjusting step of adjusting, in a CD direction, a position of an end portion in the CD direction in the continuous sheet, the CD direction intersecting the direction of transport in the continuous sheet, the position adjusting step being performed, by a position adjustment device, at an adjustment processing position in the direction of transport; and a detecting step of detecting a trace of the processing left on the continuous sheet, at a detection processing position downstream in the direction of transport from the processing position and the adjustment processing position, and outputting a detection result, the supplying step including adjusting, based on the detection result, a tension in the direction of transport of the continuous sheet at a time of being transferred to the processing section, at a position upstream in the direction of transport from the processing position.
According to such a method for manufacturing a sheet-like member associated with an absorbent article, a trace of the processing left on the continuous sheet is detected at the detection processing position located downstream in the direction of transport from the abovementioned processing position and adjustment processing position, and the tension of the continuous sheet at the time of being transferred to the processing section is adjusted based on the detection result. Accordingly, through contraction or stretch in the direction of transport of the continuous sheet by virtue of the adjustment of the tension, it is possible to locate the position of the processing of the continuous sheet close to the appropriate position. This makes it possible to reduce the amount of deviation of the position of the processing from the appropriate position in the direction of transport which may be caused by adjusting, in the CD direction, the position of the end portion in the CD-direction of the continuous sheet.

In such a method for manufacturing a sheet-like member associated with an absorbent article, it is desirable that when the detection result indicates that the trace of the processing is located downstream in the direction of transport from a target position on the continuous sheet, the tension is increased, and when the detection result indicates that the trace of the processing is located upstream in the direction of transport from the target position, the tension is decreased.

According to such a method for manufacturing a sheet-like member associated with an absorbent article, when the detection result indicates that the position of the trace of the processing is located downstream from the target position, the tension is increased, and when the detection result indicates that the position of the trace of the processing is located upstream from the target position, the tension is decreased. Accordingly, through contraction or stretch in the direction of transport of the continuous sheet based on such adjustment of the tension, it is possible to locate the position of the processing of the continuous sheet close to the appropriate position. This makes it possible to ensure the reduction in the amount of deviation in the direction of transport of the position of the processing from the appropriate position.

In such a method for manufacturing a sheet-like member associated with an absorbent article, the adjustment processing position is located downstream in the direction of transport from the processing position.

According to such a method for manufacturing a sheet-like member associated with an absorbent article, since the adjustment processing position is located downstream in the direction of transport from the processing position, it is possible to transfer the continuous sheet having been subjected to the position adjustment in the CD direction to the next step faster than a case where the positional relationship between the adjustment processing position and the processing position is opposite. Accordingly, in the next step, it is possible to perform the processing of the next step while the position of the end portion in the CD-direction is kept almost unchanged from the position of the end portion that has been adjusted in the CD direction. This makes it possible to improve the positional accuracy in the CD direction of the processing.

In such a method for manufacturing a sheet-like member associated with an absorbent article, it is desirable that when the continuous sheet is defined as a first continuous sheet, a second continuous sheet different from the first continuous sheet is transported along a continuous direction in which the second continuous sheet continues, the continuous direction conforming to the direction of transport, while a bonding device layers and bonds the first continuous sheet and the second continuous sheet together at a gathering position downstream in the direction of transport from the adjustment processing position, and the detection processing position is located downstream in the direction of transport from the gathering position.

According to such a method for manufacturing a sheet-like member associated with an absorbent article, it is possible to arrange the adjustment processing position close to the gathering position. This makes it possible to bond the first continuous sheet to the second continuous sheet in a state where the position of the end portion in the CD-direction of the first continuous sheet is kept almost unchanged from the position of the end portion that has been adjusted in the CD direction. The details are as follows.

In order to bond the first continuous sheet and the second continuous sheet together in the appropriate relative positional relationship with respect to the direction of transport, it is theoretically preferable to set the abovementioned detection processing position at the abovementioned gathering position. That is, this is ideal when the trace of the processing is detected at the gathering position, since the position of the processing of the first continuous sheet is likely to match the appropriate position which is also appropriate for the second continuous sheet. However, the abovementioned bonding device is located at the gathering position. This makes it difficult to set the detection processing position at the gathering position. Thus, the detection processing position needs to be set upstream or downstream in the direction of transport from the gathering position. Then, for example, in the former case where the detection processing position is located upstream therefrom, the detection processing position is set between the adjustment processing position and the gathering position. However, in this case, since a space for arranging a detection sensor, which should be arranged at the detection processing position, is to be secured, the abovementioned adjustment processing position needs to be set at a position at further upstream from the gathering position. This makes it difficult to maintain the position in the CD direction of the end portion that has been adjusted at the adjustment processing position until it arrives at the gathering position. Consequently, the first continuous sheet may be bonded to the second continuous sheet while the position of the end portion in the CD-direction is greatly changed from the position of the end portion that has been adjusted. That is, the positional accuracy of the end portion in the CD-direction at the gathering position may be deteriorated. However, in this regard, when the detection processing position is set downstream in the direction of transport from the gathering position as described above, it is possible to arrange the adjustment processing position close to the gathering position without interference with the abovementioned space for arranging the detection sensor. This makes it possible to bond the first continuous sheet to the second continuous sheet while the position of the end portion in the CD-direction of the first continuous sheet is kept almost unchanged from the position of the end portion that has been adjusted.

In such a method for manufacturing a sheet-like member associated with an absorbent article, it is desirable that when the continuous sheet is defined as the first continuous sheet, the second continuous sheet different from the first continuous sheet is transported along the continuous direction in which the second continuous sheet continues, the continuous direction conforming to the direction of transport, while the bonding device layers and bonds the first continuous sheet and the second continuous sheet together at the gathering position downstream in the direction of transport from the adjustment processing position, and a stiffness in the direction of transport of the second continuous sheet is larger than a stiffness in the direction of transport of the first continuous sheet, and the method further comprises: a second supplying step of transferring and supplying the second continuous sheet to second processing section, by transporting the second continuous sheet in the direction of transport that conforms to the continuous direction in which the second continuous sheet continues; a second processing step of performing a second processing onto portions in the second continuous sheet at intervals in the direction of transport, using a second processing device in the second processing section, at a second processing position located upstream in the direction of transport from the gathering position, the portions each corresponding to the absorbent article; and a second position adjusting step of adjusting a position of an end portion in the CD direction of the second continuous sheet so as to be located at a target position in the CD direction, the CD direction intersecting the direction of transport in the second continuous sheet, the second position adjusting step being performed, by a second position adjustment device, at a second adjustment processing position in the direction of transport, wherein the second supplying step includes maintaining constant a tension in the direction of transport of the second continuous sheet at a time of being transferred to the second processing section.

According to such a method for manufacturing a sheet-like member associated with an absorbent article, the second supplying step includes keeping constant the tension in the direction of transport of the second continuous sheet at the time of being transferred to the second processing section. Accordingly, the second supplying step saves the use of a device for changing the target value of the tension of the second continuous sheet. This makes it possible to reduce the facility cost.

Also, it is possible to effectively prevent change in the state of stretch of the second continuous sheet, which may be caused by unnecessary change in the target value of the tension, from adversely affecting the position of the processing in the first continuous sheet by way of the gathering position.

The reason why there is no serious issue although the tension in the direction of transport of the second continuous sheet is kept constant as described above is as follows. First of all, the stiffness in the direction of transport of the second continuous sheet is larger than the stiffness in the direction of transport of the first continuous sheet. Accordingly, the stiffness of the second continuous sheet can be set to a high value . Thus, even if the position adjustment in the CD-direction of the abovementioned end portion of the second continuous sheet is performed, the contraction or stretch in the direction of transport of the second continuous sheet can be made small. Consequently, the position of the second processing of the second continuous sheet is substantially located at the appropriate position, although the tension of the second continuous sheet at the time of being transferred to the second processing section in the second supplying step is kept constant.

In such a method for manufacturing a sheet-like member associated with an absorbent article, it is desirable that when the processing, the processing position, the processing step, the processing device, the adjustment processing position, the position adjusting step, and the position adjustment device are respectively defined as the first processing, a first processing position, a first processing step, a first processing device, a first adjustment processing position, a first position adjusting step, and a first position adjustment device, the method further comprises : a third processing step of performing third processing onto portions of the continuous sheet at intervals in the direction of transport, the third processing step being performed, by a third processing device, at a third processing position downstream in the direction of transport from the first processing position and the first adjustment processing position in the processing section, the portions each corresponding to the absorbent article; a third position adjusting step of adjusting, in the CD direction, the position of the end portion in the CD direction of the continuous sheet, using a third position adjustment device, at a third adjustment processing position downstream in the direction of transport from the first processing position and the first adjustment processing position; and a third detecting step of detecting a trace of the third processing left on the continuous sheet, at a third detection processing position downstream in the direction of transport from the third processing position and the third adjustment processing position, and outputting a third detection result, wherein the third processing device changes timing of performing the third processing based on the third detection result.

According to such a method for manufacturing a sheet-like member associated with an absorbent article, in the first processing step positioned on the upstream side, the amount of deviation of the position of the first processing in the direction of transport is reduced by the abovementioned adjustment in tension; however, in the third processing step positioned on the downstream side, the amount of deviation of the position of the third processing in the direction of transport is reduced by changing the timing of the third processing performed by the third processing device. This prevents reduction in the amount of deviation of the position of the third processing from affecting the first processing step which is positioned upstream from the third processing step. That is, it is possible to effectively prevent the position of the first processing of the first processing step positioned on the upstream side from greatly deviating from the appropriate position due to the reduction in the amount of deviation of the position of the third processing.

In such a method for manufacturing a sheet-like member associated with an absorbent article, it is desirable that the supplying step uses a feeding device that feeds the continuous sheet from a material coil, the continuous sheet is fed and transported by the feeding device in a feeding direction that conforms to an X direction in a plan view, while the continuous sheet is transported by the processing section in the direction of transport that conforms to a Y direction, the Y direction intersecting the X direction in the plan view, the direction of transport of the continuous sheet is changed from the X direction to the Y direction by running the continuous sheet about a rod-shaped transport-direction-changing member that is arranged at a position between the feeding device and the processing section in the direction of transport, and a tension adjustment device that adjusts the tension is arranged at a position between the transport-direction-changing member and the processing section in the direction of transport.

According to such a method for manufacturing a sheet-like member associated with an absorbent article, the tension adjustment device is arranged close to the processing section. This ensures that the continuous sheet whose tension has been adjusted is transferred to the processing section.

The feeding device performs feeding in the X direction as the feeding direction. Accordingly, it is possible to reduce the facility space in the Y direction for the supplying step as a whole. Consequently, the total length dimension in the Y direction of a manufacturing apparatus which performs the manufacturing method can be reduced even though the direction of transport in the processing section extends along the Y direction, thereby being able to make the manufacturing apparatus compact.

In such a method for manufacturing a sheet-like member associated with an absorbent article, it is desirable that when the tension adjustment device is defined as a downstream tension adjustment device, an upstream tension adjustment device is arranged between the feeding device and the transport-direction-changing member in the direction of transport, the upstream tension adjustment device being configured to control a feeding operation of the feeding device such that a tension of the continuous sheet fed by the feeding device is adjusted to a predetermined value.

According to such a method for manufacturing a sheet-like member associated with an absorbent article, the continuous sheet passes the transport-direction-changing member while the tension of the continuous sheet being adjusted to the predetermined value by the upstream tension adjustment device. Accordingly, even in a case where the optimal tension at the time of passing the transport-direction-changing member is different from the optimal tension at the time of transferring the continuous sheet to the processing section, such a case can be handled without serious issues.

In such a method for manufacturing a sheet-like member associated with an absorbent article, it is desirable that the supplying step uses a feeding device that feeds the continuous sheet from a material coil and includes supplying the continuous sheet to the processing section after the tension adjustment device adjusts the tension of the continuous sheet fed from the material coil, the tension adjustment device includes a dancer roll that is guided so as to be movable in a predetermined direction, a loop of the continuous sheet is formed by running the continuous sheet about the dancer roll, while an actuator applies, to the dancer roll, a load in a direction of increasing a size of the loop in the predetermined direction, the load having a size corresponding to a target value of the tension of the continuous sheet, and the target value is changed based on the detection result.

According to such a method for manufacturing a sheet-like member associated with an absorbent article, it is ensured that the tension in the direction of transport of the continuous sheet at the time of being transferred to the processing section can be adjusted based on the abovementioned detection result.

Further, an apparatus for manufacturing a sheet-like member associated with an absorbent article by using a continuous sheet, the apparatus comprising: a supplying section that transfers and supplies the continuous sheet to a processing section by transporting the continuous sheet in a direction of transport that conforms to a continuous direction in which the continuous sheet continues; a processing device that performs processing onto portions of the continuous sheet at intervals in the direction of transport, at a processing position in the direction of transport in the processing section, the portions each corresponding to the absorbent article; a position adjustment device that adjusts, in a CD direction, a position of an end portion in the CD direction of the continuous sheet, at an adjustment processing position in the direction of transport, the CD direction intersecting the direction of transport in the continuous sheet; and a detecting device that detects a trace of the processing left on the continuous sheet, at a detection processing position downstream in the direction of transport from the processing position and the adjustment processing position, and outputs a detection result, the supplying section being configured to adjust, based on the detection result, a tension in the direction of transport of the continuous sheet at a time of being transferred to the processing section, at a position upstream in the direction of transport from the processing position; wherein the adjustment processing position is located downstream in the direction of transport from the processing position.

According to such an apparatus for manufacturing a sheet-like member associated with absorbent article, it is possible to achieve effects similar to those of the foregoing manufacturing method.

First Embodiment

A method and an apparatus for manufacturing a sheet-like member associated with an absorbent article according to a first embodiment are used in the manufacturing line LM of a disposable diaper 1. That is, the method and the apparatus of the first embodiment are applied to manufacturing of a disposable diaper 1, which is an example of an absorbent article.

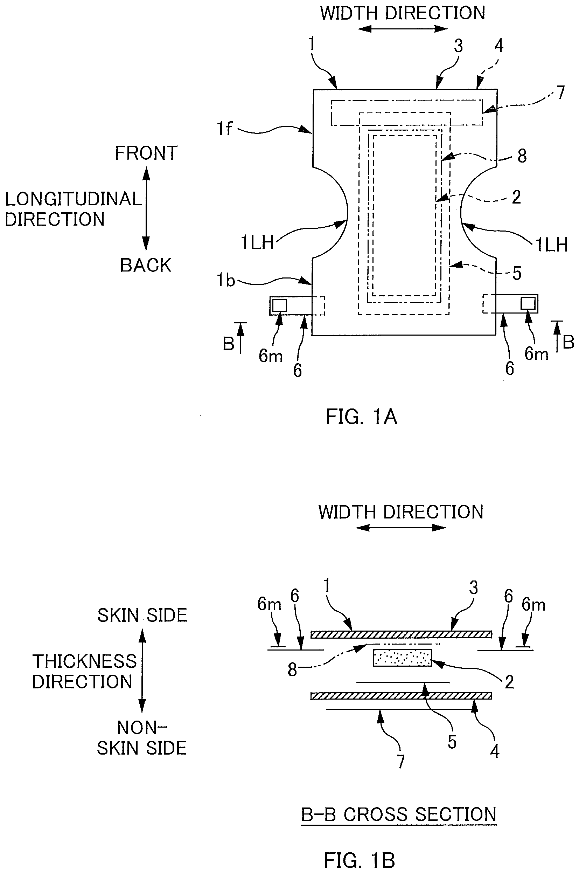

FIG. 1A is a schematic plan view of an example of the disposable diaper 1 in an unfolded state. FIG. 1B is a cross-sectional view taken along line B-B in FIG. 1A.

The diaper 1 is a so-called tape-type diaper 1. That is, it is a diaper which is put on a wearer using a fastening-tape member 6 and a target tape 7. In an unfolded state shown in FIG. 1A in which the diaper 1 is opened by disengaging the fastening-tape member 6 and the target tape 7, the diaper 1 has three directions perpendicular to one another: a longitudinal direction, a width direction and a thickness direction.

The diaper 1 includes: an absorbent body 2 that absorbs excrement such as urine; a liquid-permeable top sheet 3 provided on the skin side of the absorbent body 2; a back sheet 4 provided on the non-skin side of the absorbent body 2; and a liquid-impermeable leak-proof sheet 5 provided between the absorbent body 2 and the back sheet 4, preventing excrement from leaking to the non-skin side.

In the unfolded state shown in FIG. 1A, the top sheet 3 and the back sheet 4 have the same substantially hourglass shape in a plan view. Accordingly, a portion narrowing inwardly in the width direction in the substantially hourglass shape serves as leg openings 1LH and 1LH when it is worn.

When the diaper is worn, one part 1f and the other part 1b in the longitudinal direction of the unfolded diaper 1 respectively serve as a front part 1f covering wearer's front side and a back part 1b covering wearer's back side. Accordingly, in order to enable to keep the shape of the diaper 1 when being worn, the back part 1b includes a pair of fastening-tape members 6 and 6 each having a male member 6m of a hook-and-loop fastener and respectively projecting to the both sides in the width direction. The front part 1f has a target tape 7, on its non-skin-side surface, which is made of nonwoven fabric or a female member of a hook-and-loop fastener for fastening the abovementioned male member 6m when the diaper is worn.

In this example, the absorbent body 2 includes: an absorbent core made of liquid absorbent fibers such as pulp fibers and having a substantially rectangular shape in a plan view; and a liquid-permeable wrapping sheet that is made of tissue paper or nonwoven fabric and that wraps the outer circumferential surface of the absorbent core. However, it is not limited thereto.

The materials of the top sheet 3 and the back sheet 4 can be exemplified by nonwoven fabric containing thermoplastic resin fibers made of polyethylene, polypropylene, and/or the like, and the material of the leak-proof sheet 5 can be exemplified by a thermoplastic resin film made of polyethylene and/or the like. However, it is not limited thereto. For example, as for materials of the top sheet 3 and the back sheet 4, a material may be employed as long as it has an appropriate stretchability in the longitudinal direction.

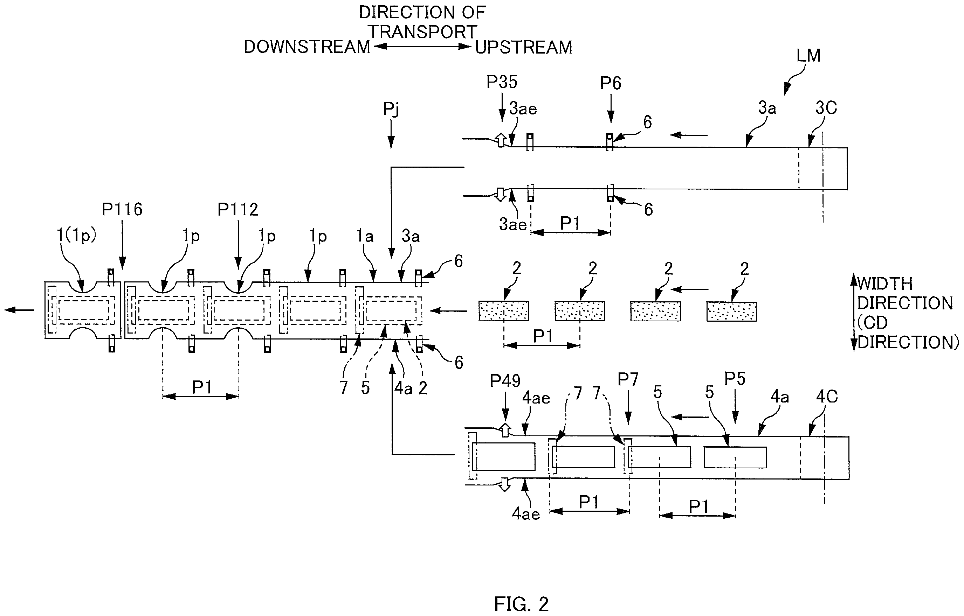

FIG. 2 is a schematic plan view illustrating a process of production of the diaper 1.

The top sheet 3 and the back sheet 4 are brought into the manufacturing line LM in the form of respective material coils 3C and 4C in which continuous sheets 3a and 4a serving as the materials are wound in a coil-like manner.

First, the continuous sheet 3a of the top sheet which has been fed from the material coil 3C is transported in the direction of transport, which is a continuous direction in which the continuous sheet 3a continues. At a predetermined position P6 in the direction of transport, a pair of the fastening-tape members 6 and 6 arranged in the width direction is bonded onto the non-skin-side surface of the continuous sheet 3a at intervals of a product pitch P1 in the direction of transport. After such bonding, at a predetermined position P35 in the direction of transport, end portions 3ae and 3ae in the width direction of the continuous sheet 3a are adjusted to be located at the target positions in the CD direction, respectively. Then, the continuous sheet 3a in the adjusted state is transported to the gathering position Pj where the continuous sheet 3a and the continuous sheet 4a of the back sheet are gathered.

Also, the continuous sheet 4a of the back sheet is fed from the other material coil 4C and is transported in the direction of transport, which is a continuous direction in which the continuous sheet 4a continues. During the transportation, at a predetermined position P5 in the direction of transport, a cutform sheet of the leak-proof sheet 5 is bonded onto the skin-side surface of the continuous sheet 4a at intervals of the product pitch P1 in the direction of transport. At a predetermined position P7 downstream of the position P5, a cutform piece of the target tape 7 is bonded onto the non-skin-side surface of the continuous sheet 4a at intervals of the product pitch P1 in the direction of transport. In addition, after such bonding, at a predetermined position P49 in the direction of transport, end portions 4ae and 4ae in the width direction of the continuous sheet 4a are adjusted to be located at the target positions in the CD direction, respectively. Then, the continuous sheet 4a in the adjusted state is transported to the abovementioned gathering position Pj.

At the gathering position Pj, the absorbent body 2 also meets . Specifically, a plurality of the absorbent bodies 2, 2... are transported toward the gathering position Pj while being aligned at the product pitches P1 in the direction of transport. At the gathering position Pj, the continuous sheet 3a of the top sheet meets the absorbent body 2 from the skin side of the absorbent body 2. And, the continuous sheet 4a of the back sheet meets the absorbent body 2 from the non-skin side of the absorbent body 2. Accordingly, these three elements are bonded to be integrated with adhesive, producing the substrate 1a of the diaper. Specifically, in the substrate 1a of the diaper, parts 1p each of which is finally to be a diaper 1 are aligned continuously at the product pitches P1 in the direction of transport.

The substrate 1a of the diaper is subsequently transported in the direction of transport. At a predetermined position P112 in the direction of transport, the widthwise ends of the substrate 1a are cut out at the product pitches P1 in the direction of transport. Accordingly, the parts 1p each of which is finally to be a diaper each becomes in the abovementioned substantially hourglass shape in a plan view. At a position P116 downstream therefrom, the substrate 1a is cut at each product pitch P1 in the direction of transport. Accordingly, the most downstream part 1p of the substrate 1a is cut and separated, thereby producing a diaper 1.

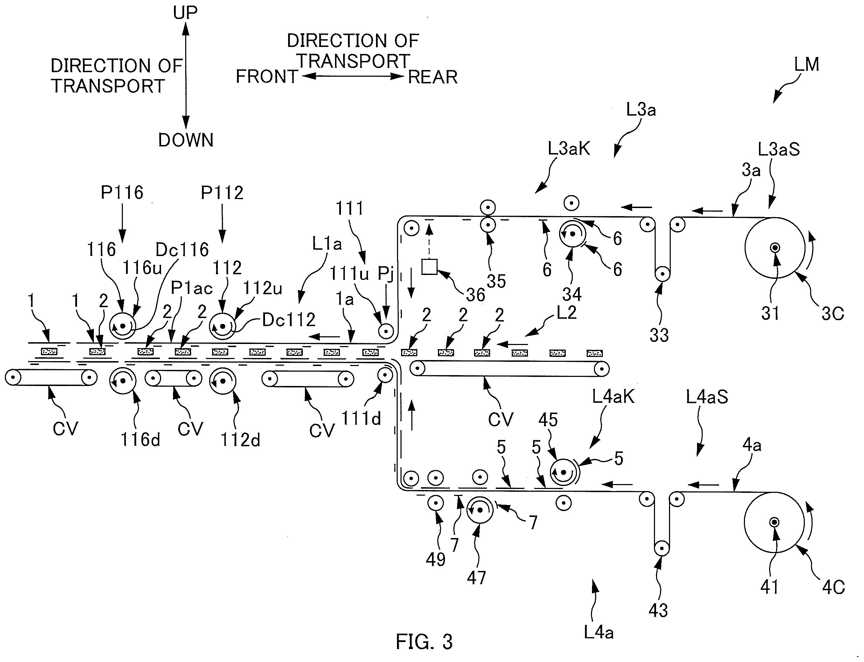

FIG. 3 is a schematic side view of the manufacturing line LM of the diaper 1.

The manufacturing line LM includes: a top-sheet processing system L3a which processes the continuous sheet 3a of the top sheet; a back-sheet processing system L4a which processes the continuous sheet 4a of the back sheet; an absorbent-body producing system L2 which produces the absorbent body 2; and a substrate processing system L1a which processes the substrate 1a of a diaper. The top-sheet processing system L3a, the back-sheet processing system L4a and the absorbent-body producing system L2 are gathered at the abovementioned gathering position Pj. Downstream from the gathering position Pj, the abovementioned substrate processing system L1a is connected.

In the substrate processing system L1a, processing the substrate 1a of the diaper is sometimes substantially equivalent to processing the continuous sheet 3a of the top sheet or processing the continuous sheet 4a of the back sheet. Accordingly, it can be said that the top-sheet processing system L3a and the back-sheet processing system L4a do not end at the gathering position Pj but continuously exist in the substrate processing system L1a. Thus, in a narrower sense, the top-sheet processing system L3a and the back-sheet processing system L4a both end at the gathering position Pj, however, in a wider sense, the top-sheet processing system L3a and the back-sheet processing system L4a do not end at the gathering position Pj but both exist extending in the substrate processing system L1a.

In the systems L3a, L4a, L2 and L1a, appropriate transport devices, such as belt conveyers CV, transport rollers, and the like, are arranged. Accordingly, unless otherwise stated, these transport devices transport ones to be transported (e.g., the continuous sheets 3a, 4a and the substrate 1a, etc.) in the direction of transport. The transport operation of such a transport device is based on a synchronization signal which will be described later. That is, each transport device is controlled in its position and transport speed so that the ones to be transported (e.g., the continuous sheets 3a and 4a, the substrate 1a, etc.) are transported at the product pitches P1 in the direction of transport each time a unit signal of the synchronization signal is transmitted. The belt conveyer CV is exemplified by an ordinary belt conveyor having an endless belt which is driven to be rotated and which serves as a transport surface, and is exemplified by a suction belt conveyor having an endless belt whose outer circumferential surface has suction function.

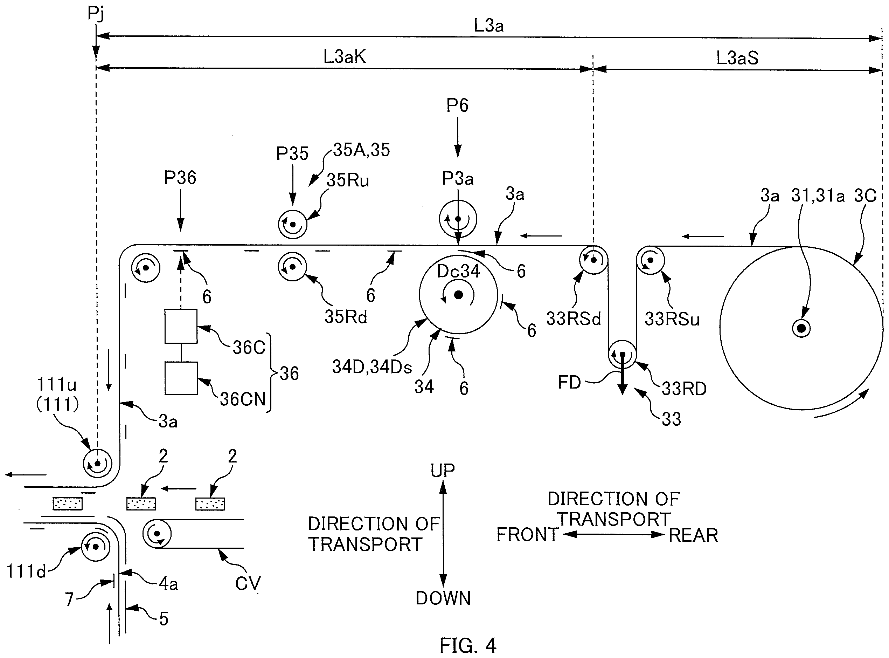

In the manufacturing line LM, the width direction of the manufacturing line LM is defined as the CD direction (a direction penetrating the paper plane of FIG. 4). In this example, the CD direction is oriented in the horizontal direction. Thus, hereinafter, the CD direction is also referred to as the "left-right direction". However, the CD direction is not limited to the horizontal direction. In this example, two directions perpendicular to the CD direction are defined as a vertical up-down direction and a horizontal front-rear direction. Accordingly, each of the directions in which the continuous sheets 3a and 4a of the top sheet and the back sheet are transported is oriented in a direction specified by the up-down direction and the front-rear direction, depending on a position in the direction of transport. The width directions of the continuous sheets 3a and 4a of the top sheet and the back sheet are each parallel to the CD direction. Directions perpendicular to the CD direction and the direction of transport are defined as Z-direction, and the Z-direction is parallel to the thickness directions of the continuous sheets 3a and 4a of the top sheet and the back sheet.

<<< Top-Sheet Processing System L3a >>>

FIG. 4 is a schematic side view of the top-sheet processing system L3a.

The top-sheet processing system L3a includes : a top-sheet supply line L3aS (corresponding to a supplying section) ; and a top-sheet processing line L3aK (corresponding to a processing section) arranged downstream from the supply line L3aS in the direction of transport. In the top-sheet supply line L3aS, the continuous sheet 3a of the top sheet (corresponding to a continuous sheet and a first continuous sheet) is fed and supplied from the material coil 3C (corresponding to a supplying step). In the top-sheet processing line L3aK, a certain process (corresponding to a processing and a first processing) is performed to the continuous sheet 3a of the top sheet which is supplied from the top-sheet supply line L3aS and transported in the direction of transport (corresponding to a processing step and a first processing step) .

(1) Top-Sheet Supply Line L3aS

The top-sheet supply line L3aS includes: a feeding device 31 for feeding the continuous sheet 3a of the top sheet from the material coil 3C; and a tension adjustment device 33 which adjusts, to a target value, the tension (N) in the direction of transport of the continuous sheet 3a which is fed by the device 31 and is transported.

As shown in FIG. 4, the feeding device 31 includes: a rotation-shaft portion 31a extending along the CD direction; and a servomotor (not shown) serving as a drive source which drives to rotate the rotation-shaft portion 31a. The rotation-shaft portion 31a is inserted into a through hole disposed at the center of the material coil 3C. The rotation-shaft portion 31a is driven and rotate by the servomotor while supporting the material coil 3C, thereby feeding the continuous sheet 3a of the top sheet from the material coil 3C.

The tension adjustment device 33 is a device for adjusting the tension (N) of the continuous sheet 3a of the top sheet to the predetermined target value as described above, and in this example, a dancer-roll type one is employed. That is, the tension adjustment device 33 includes: a pair of stationary rolls 33RSu and 33RSd; a dancer roll 33RD; a measuring device (not shown); and a control section (not shown). The stationary rolls 33RSu and 33RSd in a pair are arranged at respective fixed positions so as to be rotatable about the rotation shafts extending along the CD direction. The dancer roll 33RD is guided so as to be movable in a predetermined direction (the up-down direction in Fig. 4) at a position between the pair of stationary rolls 33RSu and 33RSd while being rotatable about the rotation shaft extending along the CD direction. The measuring device is a device, such as an encoder and/or the like, which detects the position of the dancer roll 33RD in the abovementioned predetermined direction and outputs positional information. The control section, such as a sequencer, a computer, and/or the like, is a section to which the abovementioned positional information is inputted. An appropriate actuator, such as an air cylinder and/or the like (not shown), applies a load FD to the dancer roll 33RD in a direction of increasing the size of a loop, which will be described later, in the abovementioned predetermined direction, the load FD corresponding to a target value of the tension of the continuous sheet 3a. Further, the continuous sheet 3a of the top sheet is run about the pair of stationary rolls 33RSu and 33RSd and the dancer roll 33RD, thereby forming a loop of the continuous sheet 3a, as well as the abovementioned control section changes the feeding speed (mpm) of the rotation-shaft portion 31a based on the abovementioned inputted positional information so that the size of the loop is constant. For example, when the tension is larger than the target value, the loop becomes smaller, and thus the instruction value of the feeding speed is corrected in a direction of being increased so that the size of the loop is increased. In contrast, when the tension is smaller than the target value, the loop becomes larger, and thus the instruction value of the feeding speed is corrected in a direction of being decreased so that the size of the loop is decreased. Such correction is repeatedly made at a predetermined control cycle. Consequently, adjustment is made such that the tension of the continuous sheet 3a at the position of the dancer roll 33RD is the abovementioned target value. It should be noted that, when the target value needs to be changed, this is implemented by controlling the abovementioned actuator so that the size of the abovementioned load FD is changed.

(2) Top-Sheet Processing Line L3aK

As shown in Fig. 4, in the top-sheet processing line L3aK, the pair of fastening-tape members 6 and 6 arranged side-by-side in the CD direction is bonded onto the continuous sheet 3a at the product pitch P1 in the direction of transport, as a processing of the continuous sheet 3a of the top sheet performed by a fastening-tape-member bonding device 34 (corresponding to a processing device or a first processing device) at the predetermined position P6 in the direction of transport. After such bonding, a CD-direction position adjustment device 35 adjusts the positions in the CD direction of the end portions 3ae and 3ae in the CD-direction of the continuous sheet 3a respectively (corresponding to a position adjusting step or a first position adjusting step) at the predetermined position P35 downstream in the direction of transport from the abovementioned predetermined position P6. Consequently, the continuous sheet 3a is transferred to the gathering position Pj in an appropriate state in which the end portions 3ae and 3ae are substantially located at the target positions in the CD direction, respectively. Hereinafter, the fastening-tape-member bonding device 34 and the CD-direction position adjustment device 35 will be described.

(A) Fastening-tape-member bonding device 34

The fastening-tape-member bonding device 34 includes a rotating drum 34D and a servomotor (not shown) . The rotating drum 34D is rotatable about a rotation shaft extending along the CD direction while its outer circumferential surface 34Ds facing the transport path of the continuous sheet 3a of the top sheet. The servomotor serves as a drive source which drives to rotate the rotating drum 34D. Then, on the outer circumferential surface 34Ds of the rotating drum 34D, a suction force is caused by suction, and thus a pair of fastening-tape members 6 and 6 positioned side-by-side in the CD direction are held on the outer circumferential surface 34Ds in a state of being arranged side-by-side at the product pitches P1 in the direction-of-rotation Dc34. Accordingly, due to the rotation of the rotating drum 34D, each pair of fastening-tape members 6 and 6 is sent toward and passes through a facing position P3a (the same position as that of the aforementioned predetermined position P6 and corresponding to a "processing position" and a "first processing position") at which the pair of fastening-tape members 6 faces the continuous sheet 3a, and the pair of fastening-tape members 6 and 6 is bonded to the continuous sheet 3a with adhesive. Thus, the pair of fastening-tape members 6 and 6 is transferred from the outer circumferential surface 34Ds of the rotating drum 34D to the continuous sheet 3a.

The rotation operation of the rotating drum 34D is basically operated according to a synchronization signal. The synchronization signal is a signal obtained by repeated output of a unit signal, and unit signals are rotational angle signals that correspond to rotational angle values of 0° to 360°. In the processing systems L3a, L4a, L2 and L1a, the devices 45, 47, 112 and 116 to be described later including the bonding device 34 have respective individual processing operations that constitute a pattern that is repeatedly performed for parts 1p, which will be the diapers 1, in the continuous sheets 3a and 4a. These individual processing operations and the unit signal are associated with each other in one-to-one correspondence through controlling the positions and speeds. For example, in the bonding device 34, its individual operation corresponds to a rotation operation for the product pitch P1. That is, the synchronization signal is transmitted to a servomotor of the rotating drum 34D, and the rotating drum 34D is controlled in terms of position so that each time a unit signal of synchronization signal is transmitted, the rotating drum 34D rotates by a rotational angle corresponding to the product pitch P1. More specifically, the servomotor of the rotating drum 34D is controlled in terms of position so that a position at which the pair of fastening-tape members 6 and 6 positioned in the CD direction are sucked and held on the outer circumferential surface 34Ds is moved to an instruction position in the direction-of-rotation Dc34 indicated by the synchronization signal. Basically, each time a unit signal of the synchronization signal is outputted, the pair of fastening-tape members 6 and 6 which are arranged at the product pitch P1 on the outer circumferential surface 34Ds are thus transported in the direction-of-rotation Dc34 by the product pitch P1.

The foregoing synchronization signal is generated by an appropriate control section (not shown) such as a sequencer or a computer. That is, the control section includes a processor and a memory, and the memory stores in advance a program for generating the synchronization signal. The processor reads out the abovementioned program in the memory and executes it, repeatedly generating a unit signal of the synchronization signal. However, it is not limited thereto. For example, the synchronization signal may be generated by an electric circuit. Alternatively, the synchronization signal may be generated by detecting, using an appropriate detector, an individual operation to be conducted by other basic device. For example, the following configuration is also acceptable: a rotary encoder (not shown) is provided to the cutter roll 116u (see FIG. 3) of the end-cutting device 116 for cutting the substrate 1a of the diaper and producing the diaper 1, the encoder being configured to detect the rotation operation of the cutter roll 116u; and the encoder outputs the foregoing unit signal in association with the rotation operation of the cutter roll 116u each time a single diaper 1 is cut out and produced from the substrate 1a.

(B) CD-direction Position Adjustment Device 35

The CD-direction position adjustment device 35 (corresponding to a position adjustment device and a first position adjustment device) is, as described above, a device that adjusts the positions of the end portions 3ae and 3ae in the CD-direction of the continuous sheet 3a of the top sheet independently of each other such that the end portions 3ae and 3ae are located at the target positions in the CD direction, respectively. With such adjustment, the continuous sheet 3a of the top sheet establishes an appropriate relative positional relationship, in the CD direction, with the continuous sheet 4a of the back sheet and the absorbent body 2 that are to meet the continuous sheet 3a at the gathering position Pj, and is bonded to these continuous sheet 4a and absorbent body 2.

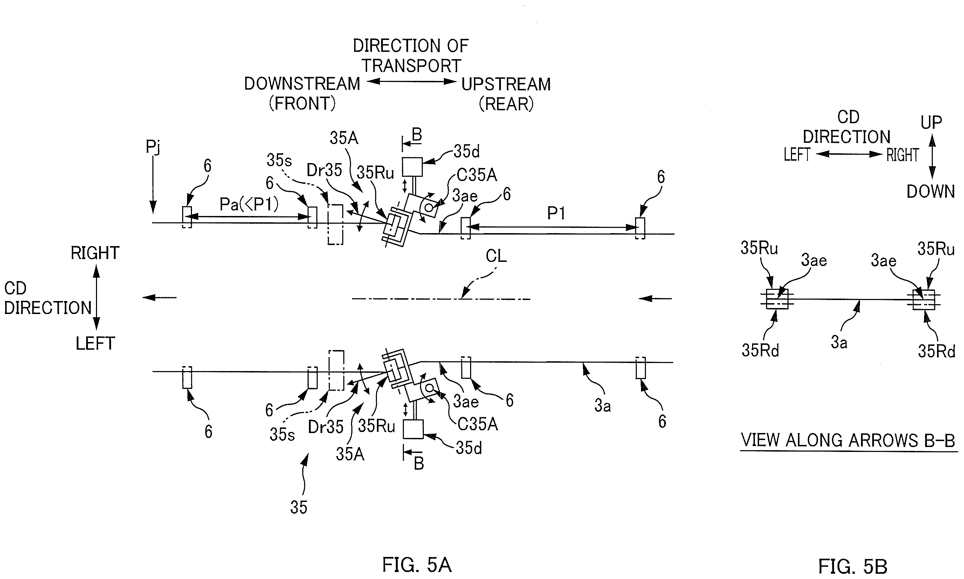

Fig. 5A is an enlarged schematic plan view illustrating a CD-direction position adjustment device 35, and Fig. 5B is a cross-sectional view taken along line B-B of Fig. 5A.

As shown in Fig. 5A, the CD-direction position adjustment device 35 includes position adjustment mechanisms 35A and 35A for adjusting the positions of the end portions 3ae and 3ae in the CD-direction of the continuous sheet 3a of the top sheet, the position adjustment mechanisms 35A and 35A being arranged at the right and left sides in the CD direction corresponding to the end portions 3ae and 3ae. These right and left position adjustment mechanisms 35A and 35A are in a mirror image relationship with each other with respect to the center line CL in the CD direction and have substantially the same basic configuration. Thus, hereinafter, only one of the position adjustment mechanisms 35A will be described.

As shown in Figs. 5A and 5B, the position adjustment mechanism 35A includes: a pair of upper and lower nip rollers 35Ru and 35Rd; an actuator 35d; a sensor 35s; and a controller not shown, for example. The pair of upper and lower nip rollers 35Ru and 35Rd are arranged and driven to rotate with their outer circumferential surfaces facing each other so as to nip the end portion 3ae in the CD-direction of the continuous sheet 3a from two sides in the thickness direction. The actuator 35d drives to turn the pair of upper and lower nip rollers 35Ru and 35Rd about a support shaft C35A in a plane including both the direction of transport and the CD direction. The sensor 35s such as a phototube, and/or the like is arranged immediately downstream of the nip rollers 35Ru and 35Rd to measure the position of the abovementioned end portion 3ae in the CD-direction and output a measurement signal. The controller controls the abovementioned actuator 35d based on the measurement signal from the sensor 35s.

In the case where the measurement signal indicates that the abovementioned end portion 3ae is located on the left side in the CD direction with respect to the target position, the controller controls the actuator 35d using an amount of control which is obtained by multiplying a deviation of the actual position indicated by the measurement signal from the target position by a predetermined gain. Accordingly, the direction of feed Dr35 in the pair of upper and lower nip rollers 35Ru and 35Rd is shifted rightward in the CD direction from the current direction of feed Dr35 by an amount corresponding to the abovementioned amount of control. On the other hand, in the case where the measurement signal indicates that the abovementioned end portion 3ae is located on the right side in the CD direction with respect to the target position, the controller similarly controls the actuator 35d using the amount of control which is obtained by multiplying a deviation of the actual position indicated by the measurement signal from the target position by a predetermined gain. Accordingly, the direction of feed Dr35 in the pair of upper and lower nip rollers 35Ru and 35Rd is shifted leftward in the CD direction from the current direction of feed Dr35 by an amount corresponding to the abovementioned amount of control. This is repeated at a predetermined control cycle, so that the end portion 3ae of the continuous sheet 3a is located at the target position in the CD direction.

However, during such position adjustment, a force in the CD direction is applied to the continuous sheet 3a of the top sheet by the position adjustment mechanisms 35A and 35A in the CD-direction position adjustment device 35, which may cause the continuous sheet 3a to contract or stretched also in the direction of transport. For example, in the case where the positions of the end portions 3ae and 3ae of the continuous sheet 3a are adjusted to increase the width dimension (dimension in the CD direction) of the continuous sheet 3a, forces directed outward in the CD direction are applied to these end portions 3ae and 3ae, respectively. In this case, the continuous sheet 3a may contract in the direction of transport by an amount corresponding to the amount of increase in the width dimension. Due to such contraction (or stretch) in the direction of transport, a pitch Pa, at which the bonding portions of the fastening-tape members 6 are arranged in the direction of transport on the continuous sheet 3a, results in a shorter pitch (or a longer pitch) relative to an appropriate pitch such as the abovementioned product pitch P1 (see Fig. 5A). When the continuous sheet 3a in such a state is fed to the gathering position Pj, the continuous sheet 3a is bonded to the continuous sheet 4a of the back sheet and the absorbent body 2 at the gathering position Pj in a state where the position of the bonding portion of the fastening-tape member 6 on the continuous sheet 3a deviates from the appropriate position (hereinafter referred to as a target position) in the direction of transport. This may deteriorate the dimensional accuracy of the diaper 1.

In the first embodiment, the bonding portion (corresponding to a trace of a processing) of the fastening-tape member 6 on the continuous sheet 3a is detected (corresponding to a detecting step), and the target value of the abovementioned tension (N) associated with the tension adjustment device 33 on the top-sheet supplying line L3aS is changed based on the detection result. That is, the tension (N) for feeding the continuous sheet 3a to the top-sheet processing line L3aK is adjusted. This reduces the amount of deviation of the abovementioned bonding portion on the continuous sheet 3a from the target position.

For example, in the case where the detection result indicates that the position of the bonding portion of the fastening-tape member 6 is located downstream in the direction of transport from the target position on the continuous sheet 3a, the target value of the tension is increased to increase the tension (N) when the continuous sheet 3a of the top sheet is fed to the top-sheet processing line L3aK. Then, thereafter, the bonding portion on the continuous sheet 3a is moved relatively upstream in the direction of transport, resulting in reduction in the amount of deviation of the position of the bonding portion from the target position. On the other hand, in the case where the detection result indicates that the position of the bonding portion is located upstream in the direction of transport from the target position, the target value of the tension is decreased to decrease the tension (N) for feeding. Then, thereafter, the bonding portion on the continuous sheet 3a is moved relatively downstream in the direction of transport, resulting in reduction in the amount of deviation in the direction of transport of the position of the bonding portion from the target position.

Such a process of reducing the amount of deviation of the position of the bonding portion from the target position is implemented using a detecting device 36 including an imaging device 36C such as a CCD camera and/or the like and a control section 36CN such as a computer and/or the like, shown in Fig. 4, as follows.

First, in this example, the imaging device 36C images the continuous sheet 3a and generates image data of the continuous sheet 3a, as shown in Fig. 4, at a position P36 in the direction of transport (hereinafter referred to as an imaging position P36 and corresponding to a "detection processing position") between the abovementioned gathering position Pj and the arrangement position P35 (the same position as the abovementioned predetermined position P35 and corresponding to an "adjustment processing position" and a "first adjustment processing position") of the nip rollers 35Ru and 35Rd of the CD-direction position adjustment device 35. Here, the imaging device 36C receives the abovementioned synchronization signal. Accordingly, the imaging operation is performed each time the rotational angle value of the synchronization signal matches the defined rotational angle value that is prestored in a memory of the imaging device 36C. In the imaging operation, a captured image has a field of view (an angle of view) being capable of including at least one pair of the fastening-tape members 6 and 6 located in the CD direction. That is, in this example, both of the fastening-tape members 6 and 6 in a pair in the CD direction are the imaging target; however, this is not limited thereto. That is, only one of the fastening-tape members 6 and 6 in a pair may be the imaging target.

Then, the control section 36CN analyzes the image data transmitted from the imaging device 36C, thereby obtaining information on the amount of deviation of the position of the bonding portion from the target position on the continuous sheet 3a of the top sheet. For example, the control section 36CN obtains the position of the bonding portion on the image data by means of binarization process or the like, and then calculates the amount of deviation of the position of the bonding portion from the target position on image data which is prestored in a memory of the control section 36CN. This result is taken as information on the amount of deviation of the position. The target position is a virtual position in the direction of transport set on the continuous sheet 3a based on the synchronization signal, so to say a design position. That is, the target position is such a position as to substantially guarantee that the fastening-tape member 6 is bonded onto the continuous sheet 3a at a position as designed in the diaper 1 as long as the bonding portion is located at that position. Such a position can be found in advance, as to which position on the image data the target position should be located at, by performing the imaging operation at the abovementioned defined rotational angle value in each unit signal of the synchronization signal. Thus, it is possible to prestore the information on the target position on the image data in the memory as described above.

Then, based on the information on the amount of deviation of the position, the control section 36CN changes the target value of the tension for the tension adjustment device 33. For example, in the case where the abovementioned information on the amount of deviation indicates that "the bonding portion is located downstream in the direction of transport from the target position" , the control section 36CN increases the target value of the tension so as to become greater than the current value by an amount of adjustment that is obtained by multiplying the amount of deviation by a predetermined gain. On the other hand, in the case where the information indicates "the bonding portion is located upstream in the direction of transport from the target position", the control section 36CN decreases the target value of the tension so as to become smaller than the current value by an amount of adjustment that is obtained by multiplying the amount of deviation by a predetermined gain.

Such a process is performed, for example, each time the abovementioned imaging operation is performed, that is, each time the abovementioned image data is generated. This makes it possible to reliably reduce the amount of deviation of the position of the bonding portion from the target position on the continuous sheet 3a.

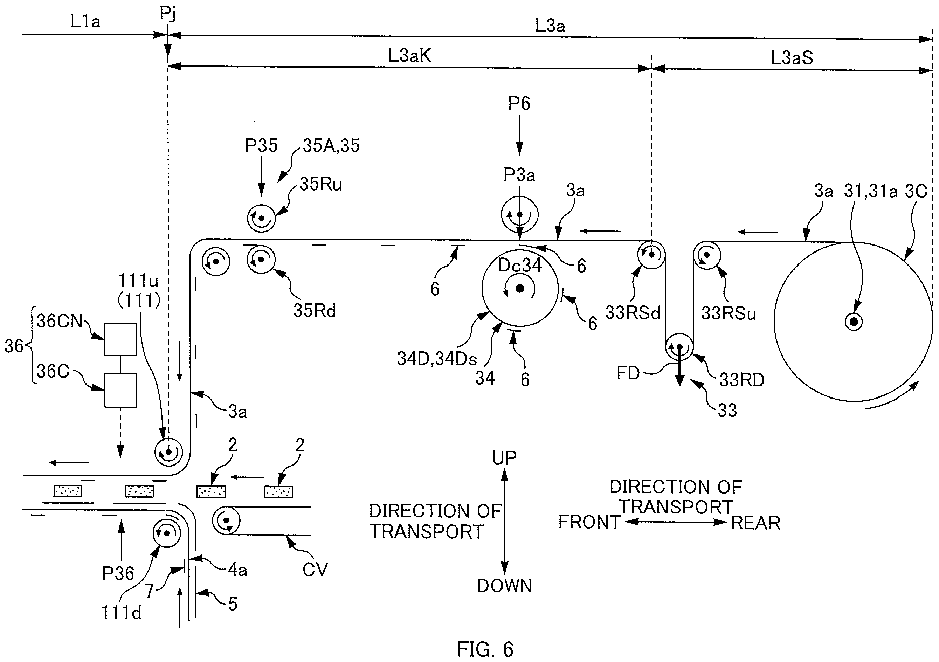

The position of the imaging position P36 is not limited to the position upstream in the direction of transport from the gathering position Pj as described above. For example, as shown in Fig. 6, the imaging position P36 may be located downstream from the gathering position Pj, that is, the imaging position P36 may be set on the transport path of the substrate processing system L1a.

When the imaging position P36 is located downstream from the gathering position Pj as such, the arrangement position P35 of the nip rollers 35Ru and 35Rd of each position adjustment mechanism 35A can be disposed close to the gathering position Pj . This makes it possible to bond the continuous sheet 3a of the top sheet to the continuous sheet 4a of the back sheet and the absorbent body 2 in a state where the end portions 3ae and 3ae in the CD-direction of the continuous sheet 3a are substantially aligned with the target positions in the CD direction. The details are as follows.

First, in order to bond the continuous sheet 3a to the continuous sheet 4a of the back sheet and the absorbent body 2 in the most appropriate relative positional relationship in the direction of transport, theoretically, it is preferable to set the abovementioned imaging position P36 at the abovementioned gathering position Pj. That is, when the bonding portion of the fastening-tape member 6 on the continuous sheet 3a is detected at the gathering position Pj, it is easy to match the position of the bonding portion of the fastening-tape member 6 to the appropriate position also for the continuous sheet 4a of the back sheet and the like, and thus the above setting would be ideal. However, a bonding device 111 is disposed at the gathering position Pj. This bonding device 111 includes, as shown in Fig. 4, the upper and lower pressing rolls 111u and 111d in a pair which rotate about respective rotation shafts extending along the CD direction. These pressing rolls 111u and 111d cooperate with each other to nip and compress three elements of the continuous sheet 3a of the top sheet, the absorbent body 2, and the continuous sheet 4a of the back sheet all together and bond and integrate these three elements.

Accordingly, since this bonding device 111 stands in the way, it is difficult to set the imaging position P36 at the gathering position Pj. As a result, the imaging position P36 needs to be set at a position deviating upstream or downstream in the direction of transport from the gathering position Pj. Here, for example, in the case where the imaging position P36 is set upstream as in an example of Fig. 4, the imaging position P36 is set between the arrangement position P35 of the nip rollers 35Ru and 35Rd and the gathering position Pj. However, in this case, the abovementioned arrangement position P35 of the nip rollers 35Ru and 35Rd needs to be set at a position distant to the further upstream side from the gathering position Pj, so as to secure a space for arranging the imaging device 36C, such as a CCD camera and/or the like, which should be arranged at the imaging position P36. This makes it difficult to maintain the position of the end portion 3ae in the CD-direction that has been adjusted by the nip rollers 35Ru and 35Rd, up to the gathering position Pj , so that the end portion 3ae is likely to deviate from the target position in the CD direction. That is, the positional accuracy of the end portion 3ae in the CD-direction is deteriorated.

However, in this regard, when the imaging position P36 is set downstream in the direction of transport from the gathering position Pj as in Fig. 6 described above, the arrangement position P35 of the nip rollers 35Ru and 35Rd can be set close to the gathering position Pj without interfering the abovementioned space for arranging the imaging device 36C. As a result, the continuous sheet 3a of the top sheet can be bonded to the continuous sheet 4a of the back sheet and the like in a state where the end portions 3ae and 3ae in the CD-direction of the continuous sheet 3a are substantially aligned with the target positions in the CD direction.

In terms of figuring out the abovementioned amount of deviation with high accuracy, it is desirable that the imaging position P36 is set at a position immediately downstream from the gathering position Pj as in an example of Fig. 6. Note that, the term "a position immediately downstream" means a given position within 2 m downstream from the gathering position Pj, more desirably, a given position within 1 m therefrom.

<<< Back-Sheet Processing System L4a>>>

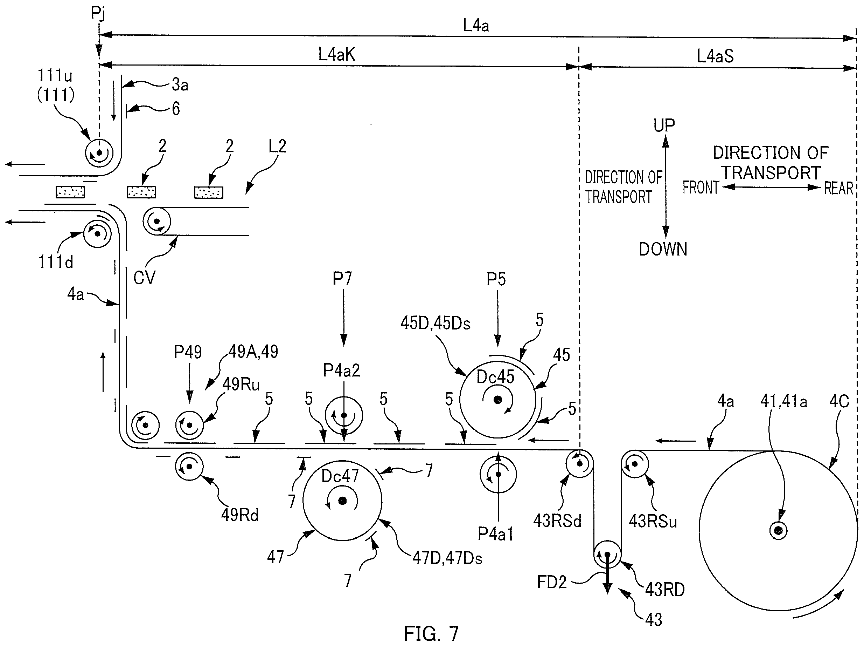

FIG. 7 is a schematic side view of the back-sheet processing system L4a.

The back-sheet processing system L4a includes: a back-sheet supply line L4aS; and a back-sheet processing line L4aK (corresponding to the second processing section) arranged downstream in the direction of transport from the supply line L4aS. In the back-sheet supply line L4aS, the continuous sheet 4a of the back sheet (corresponding to a second continuous sheet) is fed and supplied from the material coil 4C (corresponding to a second supplying step). In the back-sheet processing line L4aK, a certain process is performed to the continuous sheet 4a of the back sheet which is supplied from the back-sheet supply line L4aS and transported in the direction of transport (corresponding to a second processing and a second processing step).

(1) Back-Sheet Supply Line L4aS

The back-sheet supply line L4aS includes: a feeding device 41 for feeding the continuous sheet 4a of the back sheet from the material coil 4C; and a tension adjustment device 43 which adjusts, to a target value, the tension (N) in the direction of transport of the continuous sheet 4a that is fed by the device 41 and is transported.

As shown in FIG. 7, the feeding device 41 includes: a rotation-shaft portion 41a extending along the CD direction; and a servomotor (not shown) serving as a drive source which drives to rotate the rotation-shaft portion 41a. The rotation-shaft portion 41a is inserted into a through hole disposed at the center of the material coil 4C. The rotation-shaft portion 41a is driven to rotate by the servomotor while supporting the material coil 4C, thereby feeding the continuous sheet 4a of the back sheet from the material coil 4C.

The tension adjustment device 43 is a device for adjusting the tension (N) of the continuous sheet 4a of the back sheet to the predetermined target value; in this example, a dancer-roll type one is employed as in the case of the aforementioned top-sheet supply line L3aS. That is, the tension adjustment device 43 includes: a pair of stationary rolls 43RSu and 43RSd; a dancer roll 43RD; an appropriate actuator (not shown) ; a measuring device (not shown); and a control section (not shown). The stationary rolls 43RSu and 43RSd in a pair are arranged at respective fixed positions so as to be rotatable about the rotation shafts extending along the CD direction. The dancer roll 43RD is guided so as to be movable in a predetermined direction (the up-down direction in FIG. 7) at a position between the pair of stationary rolls 43RSu and 43RSd while being rotatable about the rotation shaft extending along the CD direction. The appropriate actuator, such as an air cylinder and/or the like, applies a load FD2 to the dancer roll 43RD in a direction of increasing the size of a loop, which will be described later, in the abovementioned predetermined direction, the load FD2 corresponding to a target value of the tension of the continuous sheet 4a. The measuring device is a device, such as an encoder and/or the like, which detects the position of the dancer roll 43RD in the abovementioned predetermined direction and outputs positional information. The control section is a section to which the abovementioned positional information is inputted. The continuous sheet 4a of the back sheet is run about the pair of stationary rolls 43RSu and 43RSd and the dancer roll 43RD, thereby forming a loop of the continuous sheet 4a. Thus, as in the case with the aforementioned top-sheet supply line L3aS, the abovementioned control section changes the feeding speed (mpm) of the rotation-shaft portion 41a based on the abovementioned positional information that is inputted so that the size of the loop is constant, thereby making an adjustment such that the tension of the continuous sheet 4a at the position of the dancer roll 43RD is the abovementioned target value. It should be noted that, when the target value is to be changed, this be achieved by changing the size of the abovementioned load FD2 by controlling the abovementioned actuator.

(2) Back-Sheet Processing Line L4aK

In the back-sheet processing line L4aK, as shown in Fig. 7, the plurality of cutform leak-proof sheets 5, 5... are bonded onto the continuous sheet 4a at the product pitch P1 in the direction of transport, as a processing subjected to the continuous sheet 4a of the back sheet at the predetermined position P5 in the direction of transport using a leak-proof sheet bonding device 45 (corresponding to a second processing device) . In addition, the plurality of cutform target tapes 7, 7... are bonded onto the continuous sheet 4a while being arranged side by side at the product pitch P1 in the direction of transport, as a processing subjected to the continuous sheet 4a by a target-tape bonding device 47 (corresponding to a second processing device) at the predetermined position P7 downstream in the direction of transport from the abovementioned predetermined position P5. After such bonding, a CD-direction position adjustment device 49 adjusts the positions of the end portions 4ae and 4ae in the CD-direction of the continuous sheet 4a in terms of the CD direction, respectively, (corresponding to a second position adjusting step) at the predetermined position P49 downstream in the direction of transport from the abovementioned predetermined position P7. Accordingly, the continuous sheet 4a is sent to the gathering position Pj while the end portions 4ae and 4ae are in the appropriate state in which they are substantially located at the target positions in the CD direction, respectively. Hereinafter, the leak-proof sheet bonding device 45, the target-tape bonding device 47, and the CD-direction position adjustment device 49 will be described.

(A) Leak-Proof-Sheet Bonding device 45

The leak-proof-sheet bonding device 45 includes a rotating drum 45D and a servomotor (not shown) . The rotating drum 45D is rotatable about a rotation shaft extending along the CD direction while its outer circumferential surface 45Ds facing the transport path of the continuous sheet 4a of the back sheet. The servomotor serves as a drive source which drives to rotate the rotating drum 45D. On the outer circumferential surface 45Ds of the rotating drum 45D, a suction force is caused by suction, and thus the plurality of the leak-proof sheets 5, 5... are held on the outer circumferential surface 45Ds while being arranged side-by-side at the product pitch P1 in the direction-of-rotation Dc45. Due to the rotation of the rotating drum 45D, each leak-proof sheet 5 is sent toward and passes through a facing position P4a1 (the same position as the aforementioned predetermined position P5, and corresponding to a "second processing position") which faces the continuous sheet 4a, and the leak-proof sheet 5 is bonded to the continuous sheet 4a with adhesive . Thus, the leak-proof sheet 5 is transferred from the outer circumferential surface 45Ds of the rotating drum 45D to the continuous sheet 4a.

The rotation operation of this rotating drum 45D is also basically operated according to a synchronization signal, as in the case with the aforementioned fastening-tape-member bonding device 34. That is, the servomotor of the rotating drum 45D is controlled in terms of position so that a position where the leak-proof sheet 5 is sucked and held on the outer circumferential surface 45Ds is moved to an instruction position in the direction-of-rotation Dc45 indicated by the synchronization signal. Basically, each time a unit signal of the synchronization signal is outputted, the cutform leak-proof sheets 5 which are placed at the product pitch P1 on the outer circumferential surface 45Ds are thus transported in the direction-of-rotation Dc45 by the product pitch P1.

(B) Target-Tape Bonding Device 47

The target-tape bonding device 47 also includes a rotating drum 47D and a servomotor (not shown) . The rotating drum 47D is rotatable about a rotation shaft extending along the CD direction while its outer circumferential surface 47Ds facing the transport path of the continuous sheet 4a of the back sheet. The servomotor serves as a drive source which drives to rotate the rotating drum 47D. On the outer circumferential surface 47Ds of the rotating drum 47D, a suction force is caused by suction, and thus the plurality of target tapes 7, 7... are held on the outer circumferential surface 47Ds while being arranged side-by-side at the product pitch P1 in the direction-of-rotation Dc47. Due to the rotation of the rotating drum 47D, each target tape 7 is sent toward and passes through a facing position P4a2 (the same position as the predetermined position and corresponding to a "second processing position") which faces the continuous sheet 4a, and the target tape 7 is bonded to the continuous sheet 4a with adhesive. Thus, the target tape 7 is transferred from the outer circumferential surface 47Ds of the rotating drum 47D to the continuous sheet 4a.

The rotation operation of the rotating drum 47D is basically operated according to a synchronization signal, as in the case with the abovementioned fastening-tape-member bonding device 34. That is, the servomotor of the rotating drum 47D is controlled in terms of position so that a position at which the target tape 7 is sucked and held on the outer circumferential surface 47Ds is moved to an instruction position in the direction-of-rotation Dc47 indicated by the synchronization signal. Basically, each time a unit signal of the synchronization signal is outputted, the target tapes 7 which are placed on the outer circumferential surface 47Ds at the product pitch P1 are transported in the direction-of-rotation Dc47 by the product pitch P1.

(C) CD-direction Position Adjustment Device 49

The CD-direction position adjustment device 49 (corresponding to a second position adjustment device) is, as described above, a device which adjusts the positions of the end portions 4ae and 4ae in the CD-direction of the continuous sheet 4a of the back sheet independently of each other such that the end portions 4ae and 4ae are located at the target positions in the CD direction, respectively. With such adjustment, the continuous sheet 4a of the back sheet establishes an appropriate relative positional relationship, in the CD direction, with the continuous sheet 3a of the top sheet and the absorbent body 2 that are to meet the continuous sheet 4a at the gathering position Pj, and is bonded to these continuous sheet 3a and absorbent body 2.

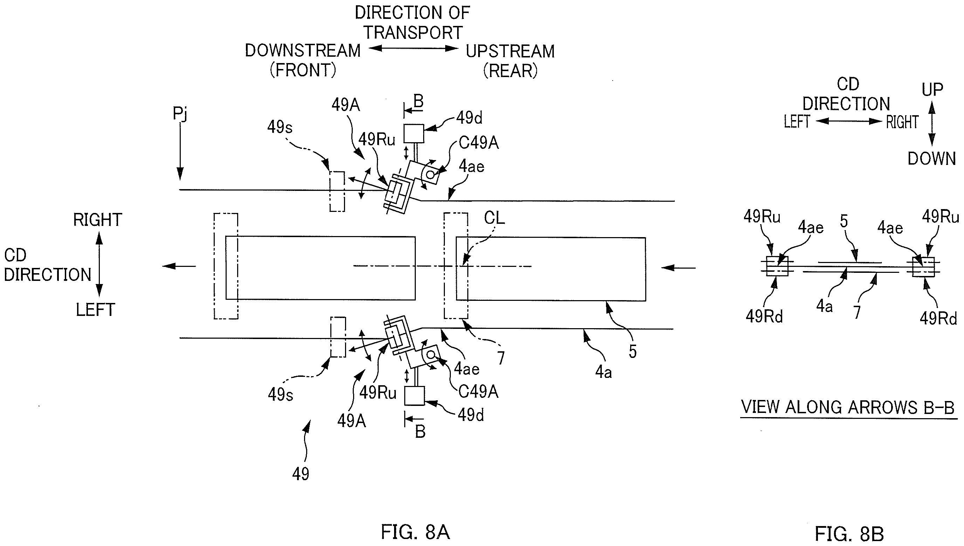

Fig. 8A is an enlarged schematic plan view illustrating a CD-direction position adjustment device 49, and Fig. 8B is a cross-sectional view taken along line B-B of Fig. 8A.

The basic configuration of the CD-direction position adjustment device 49 is substantially the same as that of the abovementioned CD-direction position adjustment device 35 provided in the top-sheet processing line L3aK. That is, the CD-direction position adjustment device 49 also includes position adjustment mechanisms 49A and 49A for adjusting the positions of the end portions 4ae and 4ae in the CD-direction of the continuous sheet 4a of the back sheet on the right and left sides in the CD direction corresponding to the end portions 4ae and 4ae. These right and left position adjustment mechanisms 49A and 49A are in a mirror image relationship with respect to the center line CL of the CD direction and have substantially the same basic configuration. The position adjustment mechanisms 49A each include: a pair of upper and lower nip rollers 49Ru and 49Rd; an actuator 49d; a sensor 49s; and a controller (not shown), for example. The pair of upper and lower nip rollers 49Ru and 49Rd are arranged and driven to rotate with their outer circumferential surfaces facing each other so as to nip the end portion 4ae in the CD-direction of the continuous sheet 4a from two sides in the thickness direction. The actuator 49d drives to turn the pair of upper and lower nip rollers 49Ru and 49Rd about a support shaft C49A in a plane including both the direction of transport and the CD direction. The sensor 49s such as a phototube and/or the like is arranged immediately downstream of the nip rollers 49Ru and 49Rd to measure the position of the abovementioned end portion 4ae in the CD-direction and output a measurement signal. The controller controls the abovementioned actuator 49d based on the measurement signal from the sensor 49s. The configurations of the elements 49Ru, 49Rd, 49d, and 49s and their positional adjustment operations are substantially the same as those of the abovementioned CD-direction position adjustment device 35 in the top-sheet processing line L3aK. Thus, further descriptions thereof are omitted.

In this example, SMS (spunbond/meltblown/spunbond) nonwoven fabric is used as a material of the continuous sheet 4a of the back sheet, and airthrough nonwoven fabric is used as a material of the abovementioned continuous sheet 3a of the top sheet. Accordingly, the stiffness in the direction of transport of the continuous sheet 4a of the back sheet is thus larger than the stiffness in the direction of transport of the continuous sheet 3a of the top sheet, and thus the stiffness of the continuous sheet 4a of the back sheet is set to a high value. For example, the stiffness is set to a value equal to or larger than 2.7 (N/25 mm), desirably a value equal to or larger than 3.5 (N/25 mm), more desirably a value equal to or larger than 4.5 (N/25 mm), and further desirably a value equal to or larger than 5.5 (N/25 mm).

Accordingly, in this case, even though the CD-direction position adjustment device 49 makes the abovementioned adjustment to the positions in the CD-direction of the continuous sheet 4a of the back sheet, the continuous sheet 4a is unlikely to contract or stretch in the direction of transport . Thus, even though the tension (N) of the continuous sheet 4a of the back sheet when transferring the continuous sheet 4a from the back-sheet supply line L4aS to the back-sheet processing line L4aK is kept constant, the pitch at which the bonding portions of the leak-proof sheets 5 are arranged in the direction of transport and the pitch at which the bonding portions of the target tapes 7 are arranged in the direction of transport on the continuous sheet 4a can be maintained at the abovementioned product pitch P1 which is substantially an appropriate pitch, thereby being able to locate the positions of the bonding portions substantially at the appropriate positions, respectively.

Accordingly, in this example, the tension adjustment device 43 in the back-sheet supply line L4aS in Fig. 7 maintains the target value of the tension at a constant value without changing the target value. This makes it possible to omit the detecting device 36, as shown in Fig. 7, which is provided in the abovementioned top-sheet processing system L3a in Fig. 4, thereby being able to reduce the facility cost. Further, it is possible to effectively prevent such an adverse effect and the like that the position of the bonding portion of the fastening-tape member 6 on the continuous sheet 3a of the top sheet is changed by way of the gathering position Pj due to the change in state of stretch of the continuous sheet 4a of the back sheet that may be caused by unnecessary change in the target value of the tension.

Note that the "stiffness (N/25 mm) " means a stiffness (N/25 mm) that is measured in a state where the continuous sheets 3a and 4a having a width dimension of 25 mm, serving as the measurement targets, are stretched at a stretch rate R of 2% in the direction of transport from their natural lengths, respectively. That is, it is possible to measure the abovementioned stiffness K (N/25 mm) as follows. First, a state in which specimens of the continuous sheets 3a and 4a are stretched in a direction corresponding to the direction of transport (hereinafter referred to as the longitudinal direction) at the abovementioned stretch rate R of 2% is defined as the initial state. The dimensions in the width direction (width dimension) orthogonal to the longitudinal direction of the specimens are set to 25 mm. A load value Fs (N) in the initial state is recorded. In addition, a load value Fe (N) when the specimens are further stretched in the longitudinal direction from the initial state until the stretch rate R reaches 5% is recorded. Then, a value obtained by substituting the abovementioned obtained values Fs and Fe into the following equation is the abovementioned stiffness (N/25 mm) .

$$K = Fe - Fs$$

Note that the "stretch rate R(%) " here is a value R (= $\Delta L/L0$) expressed in percentage, which is obtained such that a natural length L0 is subtracted from a length L in the longitudinal direction in a stretched state in the longitudinal direction to obtain a subtracted value $\Delta L$ (= L - L0) and then the subtracted value $\Delta L$ (= L - L0) is divided by the natural length L0.

<<< Absorbent-Body Producing System L2 >>>

As shown in FIG. 3, the absorbent-body producing system L2 includes a belt conveyer CV serving as a transport device. The belt conveyer CV is operated in conjunction with the abovementioned synchronization signal, thereby transporting the absorbent bodies 2, 2... in the direction of transport at the product pitch P1 to the gathering position Pj.

<<< Substrate Processing System L1a >>>

With reference to FIG. 3, the substrate processing system L1a is located downstream with respect to the gathering position Pj as mentioned above. In the substrate processing system L1a, a leg-opening cutting device 112 and an end-cutting device 116 are arranged in this order from upstream to downstream in the direction of transport. First, the cutting device 112 cuts out both side portions in the CD-direction of the substrate 1a of the diaper which is transported from the gathering position Pj, to form the pair of leg openings 1LH and 1LH in the substrate 1a, which are positioned side-by-side in the CD direction. Next, the cutting device 116 cuts the substrate 1a at a position Plac which is between the absorbent bodies 2 and 2 adjacent in the direction of transport, to separate a downstream end portion from the substrate 1a, thereby producing a diaper 1 shown in FIG. 1A.
The leg-opening cutting device 112 includes: a pair of upper and lower rolls 112u and112d provided rotatably about the rotation shaft extending along the CD direction; and servomotors (not shown) that drives to rotate the pair of rolls 112u and 112d as drive sources. The upper roll 112u is a cutter roll. That is, on the outer circumferential surface of the upper roll 112u, cutter blades (not shown) are provided at each predetermined angle in the direction-of-rotation Dc112 and the cutter blades each have a curved shape corresponding to the leg opening 1LH of the diaper 1. On the other hand, the lower roll 112d is an anvil roll whose outer circumferential surface receives the cutter blade. The rotation operations of these rolls 112u and 112d are basically operated according to the abovementioned synchronization signal. That is, basically, a servomotor is controlled in terms of position so that a position of the cutter blade is moved to an instruction position in the direction-of-rotation Dc112 indicated by the synchronization signal. Accordingly, each time a unit signal of the synchronization signal is outputted, the cutter blade rotates so as to face a pair of portions where leg openings 1LH are to be formed of the substrate 1a of the diaper, that is, so as to face portions of the substrate 1a that are located outside the absorbent body 2 in the CD direction. Consequently, leg openings 1LH and 1LH are formed in the substrate 1a.
The end-cutting device 116 includes: a pair of upper and lower rolls 116u and 116d provided so as to be rotatable about the rotation shaft extending along the CD direction; and servomotors (not shown) that drive to rotate the pair of rolls 116u and 116d as drive sources. The upper roll 116u is a cutter roll. That is, on the outer circumferential surface of the upper roll 116u, cutter blades (not shown) are provided at each predetermined angle in the direction-of-rotation Dc116, and the cutter blades each have a straight shape extending along the CD direction. On the other hand, the lower roll 116d is an anvil roll whose outer circumferential surface receives the cutter blade. The rotation operations of these rolls 116u and 116d are basically operated according to the abovementioned synchronization signal. That is, basically, a servomotor is controlled in terms of position so that a position of the cutter blade is moved to an instruction position in the direction-of-rotation Dc116 indicated by the synchronization signal. Accordingly, each time a unit signal of the synchronization signal is outputted, the cutter blade rotates so as to face a position P1ac at which cutting is performed for the substrate 1a of the diaper, that is, so as to face the position Plac between the absorbent bodies 2 and 2 adjacent in the direction of transport in the substrate 1a. Consequently, a downstream end portion is cut and separated from the substrate 1a, thereby producing a diaper 1.

=== Modified Example ===

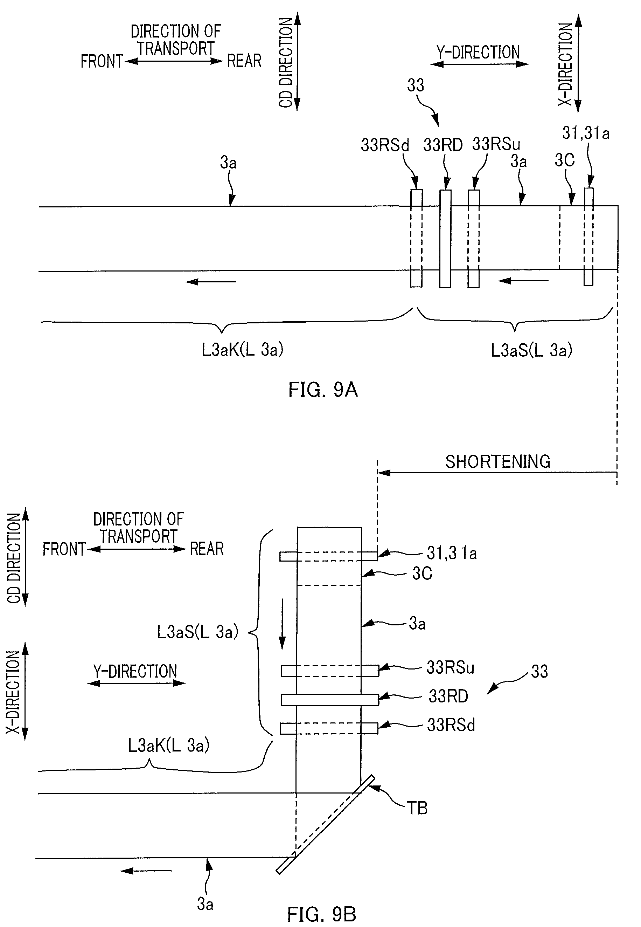

As shown in the schematic plan view of FIG. 9A, a direction along the CD direction when the manufacturing line LM is viewed in a plan view is defined as "X-direction", and a direction perpendicular to the CD direction is defined as "Y-direction". In the foregoing first embodiments, as shown in FIG. 9A, a direction of feeding of the continuous sheet 3a by the feeding device 31 in the top-sheet supply line L3aS is Y-direction, and also the direction of transporting the continuous sheet 3a in the top-sheet processing line L3aK is Y-direction in the same direction as the foregoing direction of feeding the continuous sheet 4a. However, it is not limited thereto. That is, the configuration shown in the modified example of FIG. 9B is also acceptable; that is, the direction of feeding in the top-sheet supply line L3aS may be mainly along X-direction, and the direction of transport in the top-sheet processing line L3aK may be mainly along Y-direction.

With such a configuration, as shown in FIG. 9B, the position where the rotation-shaft portion 41a of the feeding device 41 is arranged can be set at a position different from the top-sheet processing line L3aK in X-direction (CD direction). This can reduce the total length of the manufacturing line LM in Y-direction, thereby being able to downsize the manufacturing line LM.

As shown in FIG. 9B, arranging the rotation-shaft portion 31a of the feeding device 31 along Y-direction realizes that the direction of feeding of the continuous sheet 3a is oriented in X-direction (CD direction). With a turn bar TB (corresponding to a transport-direction-changing member), the direction of transport of the continuous sheet 3a can be changed from X-direction to Y-direction. The turn bar TB is, for example, a round bar having a diameter of 25.4 mm. The turn bar TB is arranged immovably and unrotatably, with the longitudinal direction thereof being inclined by 45 degrees with respect to both of X-direction and Y-direction. Accordingly, the continuous sheet 3a of the top sheet is caused to run about the turn bar TB, thereby changing the direction of transport of the continuous sheet 3a from X-direction to Y-direction.

However, provision of the turn bar TB makes it easier to cause such a trouble that, when variation in tension of the continuous sheet 4a of the top sheet occurs at the position of the turn bar TB, the continuous sheet 3a is caught by the unrotatable turn bar TB, sliding resistance between the continuous sheet 3a and the turn bar TB becomes excessively large, and/or the like. Accordingly, it is necessary to set the tension to the optimal value for allowing the sheet to run through the turn bar TB. The foregoing tension (N) may be different from an optimal tension (N) for transferring the continuous sheet 4a to the top-sheet processing line L3aK. For example, the former optimal tension may be smaller than the latter optimal tension, or vice versa.

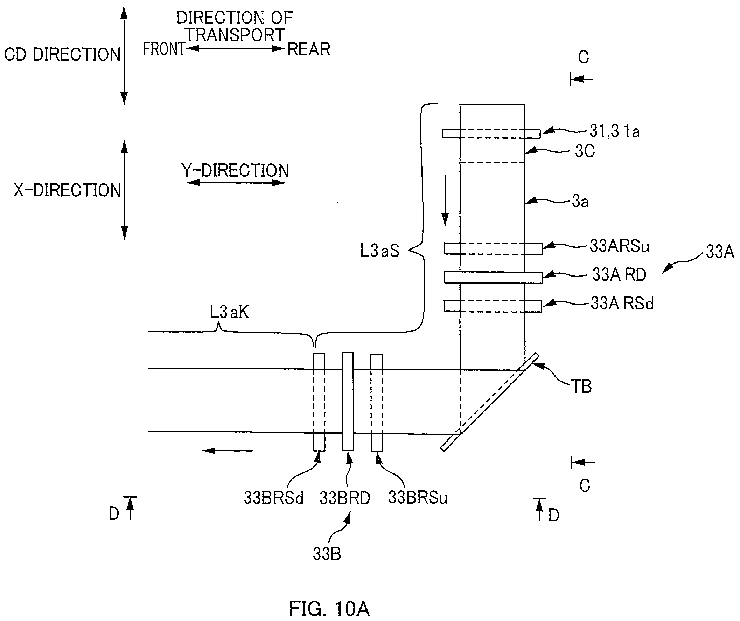

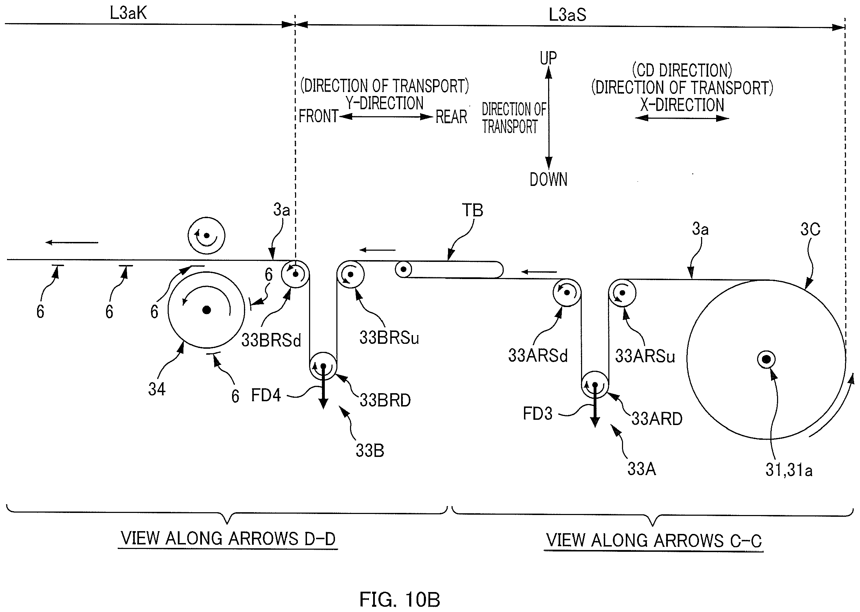

In such a case, it is preferable that two tension adjustment devices 33A and 33B are provided. FIGS. 10A and 10B are diagram showing an example thereof. FIG. 10A is a schematic plan view. FIG. 10B is a schematic side view obtained by connecting a view along arrows C-C of FIG. 10A and a view along arrows D-D of FIG. 10A are connected and illustrating them in a plane.

As shown in FIGS. 10A and 10B, in this example, one tension adjustment device 33A (corresponding to an upstream tension adjustment device) is provided between the feeding device 31 and the turn bar TB, and also another tension adjustment device 33B (corresponding to a downstream tension adjustment device) is provided between the turn bar TB and the top-sheet processing line L3aK. This makes it possible to adjust each tension individually.

As shown in FIG. 10B, the tension adjustment device 33A adjusts the tension at the position of the turn bar TB. The configuration of the tension adjustment device 33A is substantially the same as that of the abovementioned tension adjustment device 33 which is a dancer-roll type. That is, the tension adjustment device 33A includes: a pair of stationary rolls 33ARSu and 33ARSd; a dancer roll 33ARD; an appropriate actuator (not shown); a measuring device (not shown); and a control section (not shown). The stationary rolls 33ARSu and 33ARSd in a pair are arranged at respective fixed positions and provided so as to be rotatable about the rotation shaft extending along Y-direction. The dancer roll 33ARD is guided so as to be movable in a predetermined direction (up-down direction in FIG. 10B) at a position between the pair of stationary rolls 33ARSu and 33ARSd while being rotatable about the rotation shaft extending along Y-direction. The actuator, such as an air cylinder and/or the like, applies a load FD3 to the dancer roll 33ARD in a direction of increasing the size of a loop, which will be described later, in the abovementioned predetermined direction, the load FD3 corresponding to the target value (corresponding to a predetermined value) of the tension of the continuous sheet 3a. The measuring device, such as an encoder and/or the like, detects the position of the dancer roll 33ARD in the predetermined direction and outputs positional information. The control section, such as a sequencer, a computer, and/or the like, is a section to which the abovementioned positional information is inputted. The continuous sheet 3a of the top sheet is run about the pair of stationary rolls 33ARSu and 33ARSd and the dancer roll 33ARD, thereby forming a loop of the continuous sheet 3a. Accordingly, the abovementioned control section changes the feeding speed (mpm) of the rotation-shaft portion 31a based on the abovementioned inputted positional information so that the size of the loop is constant, thereby making an adjustment such that the tension (N) of the continuous sheet 3a at the position of the dancer roll 33ARD is the predetermined target value, and furthermore the tension (N) at the position of the turn bar TB is the predetermined target value.

On the other hand, as shown in FIG. 10B, the tension adjustment device 33B adjusts a tension at the time of transferring the continuous sheet 3a to the top-sheet processing line L3aK. The device 33B is also a dancer-roll type. That is, the tension adjustment device 33B includes: a pair of stationary rolls 33BRSu and 33BRSd; a dancer roll 33BRD; an appropriate actuator (not shown); a measuring device (not shown); and a control section (not shown). The stationary rolls 33BRSu and 33BRSd in a pair are arranged at respective fixed positions and provided so as to be rotatable about the rotation shaft extending along X-direction. The dancer roll 33BRD is guided so as to be movable in a predetermined direction (up-down direction in FIG. 10B) at a position between the pair of stationary rolls 33BRSu and 33BRSd while being rotatable about the rotation shaft extending along X-direction. The actuator, such as an air cylinder and/or the like, applies a load FD4 to the dancer roll 33BRD in a direction of increasing the size of a loop, which will be described later, in the abovementioned predetermined direction, the load FD4 corresponding to the target value of the tension of the continuous sheet 4a. The measuring device, such as an encoder and/or the like, detects the position of the dancer roll 33BRD in the predetermined direction and outputs positional information. The control section, such as a sequencer, a computer, and/or the like, is a section to which the abovementioned positional information is inputted. The continuous sheet 4a of the back sheet is run about the pair of stationary rolls 33BRSu and 33BRSd and the dancer roll 33BRD, thereby forming a loop of the continuous sheet 3a. Of the pair of stationary rolls 33BRSu and 33BRSd, the stationary roll 33BRSu is located upstream in the direction of transport, in other words, the stationary roll 33BRSu is located upstream in the direction of transport from the dancer roll 33BRD. The stationary roll 33BRSu serves as a driving roll (hereinafter also referred to as a stationary driving roll 33BRSu) which is rotated by a servomotor as a drive source. Accordingly, based on the abovementioned inputted positional information, the abovementioned control section changes the rotation speed (mpm) of the abovementioned stationary driving roll 33BRSu so that the size of the loop is constant, thereby making an adjustment such that the tension of the continuous sheet 3a at the position of the dancer roll 33BRD is the predetermined target value.

Further, similarly to the abovementioned first embodiment, the target value of the tension (N) for the tension adjustment device 33B is changed based on the detection result of the bonding portions of the fastening-tape members 6 and 6 in the continuous sheet 3a of the top sheet. For example, in the case where the detection result of the detecting device 36 indicates that the bonding portions of the fastening-tape members 6 and 6 are located downstream in the direction of transport with respect to the target positions in the continuous sheet 3a, the control section 36 of the detecting device 36 increases the target value of the tension. This increases the tension at the time of transferring the continuous sheet 3a of the top sheet to the top-sheet processing line L3aK. On the contrary, in the case where the detection result indicates that the bonding portion is located upstream in the direction of transport with respect to the target position, the control section 36 decreases the target value of the tension. This decreases the tension at the time of transferring.

It should be noted that, the target value can be changed by changing the magnitude of the load FD4 by controlling the actuator.

Second Embodiment

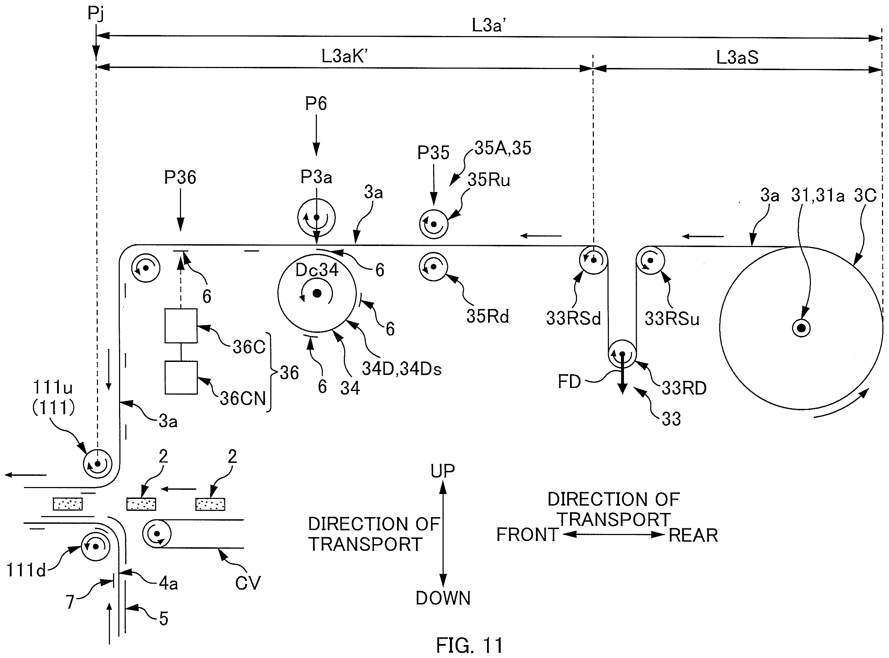

Fig. 11 is a schematic side view of a top-sheet processing line L3aK' in a top-sheet processing system L3a' according to a second embodiment.

In the abovementioned first embodiment, as shown in Fig. 4, the CD-direction position adjustment device 35 is located downstream in the direction of transport with respect to the fastening-tape-member bonding device 34; however, in the second embodiment, the order of the arrangement in the direction of transport of these two devices 34 and 35 is reversed as shown in Fig. 11. That is, the second embodiment mainly differs from the first embodiment in that the CD-direction position adjustment device 35 is arranged upstream in the direction of transport with respect to the fastening-tape-member bonding device 34. Rest of the configuration of the second embodiment is substantially the same as that of the abovementioned first embodiment. Thus, hereinafter, this difference is mainly described, and the similar configurations are denoted by the same reference signs while descriptions thereof are omitted.

In the second embodiment, the CD-direction position adjustment device 35 is arranged upstream in the direction of transport with respect to the fastening-tape-member bonding device 34 as described above. Thus, the continuous sheet 3a of the top sheet is transferred to the fastening-tape-member bonding device 34 in a state where the CD-direction position adjustment device 35 accurately aligns the end portions 3ae and 3ae in the CD-direction of the continuous sheet 3a with the target positions in the CD direction. Consequently, the fastening-tape-member bonding device 34 can bond the fastening-tape members 6 and 6 to the end portions 3ae and 3ae in the CD-direction of the continuous sheet 3a with high positional accuracy in the CD direction.

However, since the positions of the end portions 3ae and 3ae of the continuous sheet 3a are adjusted in the CD direction before the fastening-tape member 6 is bonded onto the continuous sheet 3a, there is a risk that the bonding portions of the fastening-tape members 6 in the continuous sheet 3a would be arranged in the direction of transport at a pitch different from the product pitch P1 in the direction of transport. The details are as follows.

For example, in the case where the CD-direction position adjustment device 35 adjusts the positions of the end portions 3ae and 3ae so as to increase the width dimension (CD-direction dimension) of the continuous sheet 3a, the continuous sheet 3a contracts in the direction of transport by a length corresponding to the amount of increase in the width dimension. When such a contracted continuous sheet 3a passes through the position of the bonding device 34, the bonding device 34 bonds the fastening-tape members 6 at the product pitch P1 in the direction of transport onto the continuous sheet 3a having been contracted in the direction of transport. However, in the case where the width dimension of the continuous sheet 3a is reduced in the CD direction to a design width dimension required for the bonding of the continuous sheet 4a of the back sheet, on the downstream side of the bonding device 34, the continuous sheet 3a may be stretched in the direction of transport by a length corresponding to the amount of contraction. This causes the fastening-tape members 6 to be arranged in the direction of transport at a pitch larger than the product pitch P1. Consequently, there is a risk that the continuous sheet 3a would be bonded to the continuous sheet 4a of the back sheet and the like at the gathering position Pj in a state where the position of the bonding portion of the fastening-tape member 6 deviates from the target position in the direction of transport.

However, in this regard, in the second embodiment, as in the case with the first embodiment, the imaging position P36 of the imaging device 36C is set downstream with respect to the arrangement positions P35 and P6 (P3a) of the CD-direction position adjustment device 35 and the fastening-tape-member bonding device 34 in the direction of transport, and the bonding portion of the abovementioned fastening-tape member 6 is detected at the imaging position P36. Accordingly, even when the fastening-tape members 6 are arranged in the direction of transport at a pitch larger than the product pitch P1 as described above, it is still possible to detect the bonding portions in such a state, and also it is possible to detect the bonding portions in the opposite case. Thus, based on the detection result, the tension adjustment device 33 of the top-sheet supply line L3aS can adjust the tension (N) for transferring the continuous sheet 3a to the top-sheet processing line L3aK'. This makes it possible to reduce the amount of deviation in the direction of transport of the positions of the bonding portions from the target positions.

Third Embodiment

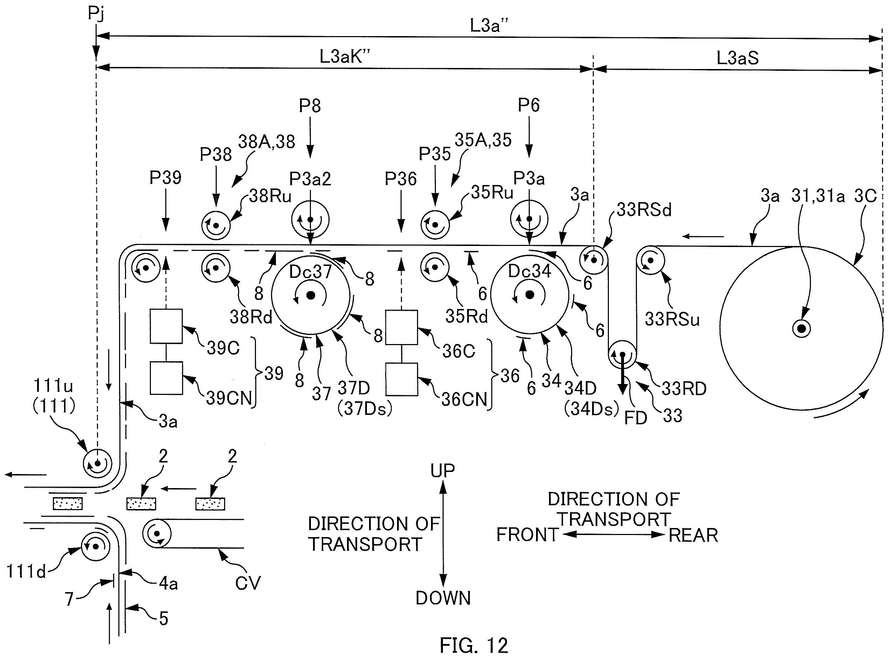

Fig. 12 is a schematic side view of a top-sheet processing line L3aK" in a top-sheet processing system L3a" of a third embodiment.

In the abovementioned first embodiment, the top-sheet processing system L3a in Fig. 4 includes only the fastening-tape-member bonding device 34 as a processing device associated with the continuous sheet 3a of the top sheet. However, in the third embodiment in Fig. 12, a cushion-sheet bonding device 37, which bonds a cushion sheet 8 onto the continuous sheet 3a, is included as a different further processing device in addition to the abovementioned bonding device 34. First of all, this point is different from the first embodiment. In addition, the third embodiment further differs from the first embodiment in that one more different CD-direction position adjustment device 38 is additionally provided correspondingly to the cushion-sheet bonding device 37. Components other than the above are substantially the same as those in the abovementioned first embodiment. Thus, hereinafter, these differences are mainly described, and the similar parts or elements are given the same reference numerals and a description thereof is omitted.

As virtually shown in Figs. 1A and 1B using a dashed double-dotted line, the cushion sheet 8 is a sheet 8 that provides cushioning characteristics to the skin side of the diaper 1. Thus, the cushion sheet 8 is interposed between the top sheet 3 and the absorbent body 2. In this example, air-through nonwoven fabric is used as a material of the cushion sheet 8; however, it is not limited thereto.

Meanwhile, as shown in Fig. 12, the cushion-sheet bonding device 37 (corresponding to a third processing device) is arranged at a predetermined position P8 downstream with respect to the arrangement positions P6 and P35 of the fastening-tape-member bonding device 34 and the CD-direction position adjustment device 35. At the predetermined position P8, the cushion-sheet bonding device 37 bonds the plurality of cutform cushion sheets 8, 8... onto the continuous sheet 3a of the top sheet while the cushion sheets 8, 8... are arranged side-by-side at intervals of the product pitch P1 in the direction of transport (corresponding to a third processing or a third processing step).

Such a cushion-sheet bonding device 37 includes a rotating drum 37D and a servomotor (not shown). The rotating drum 37D is rotatable about a rotation shaft extending along the CD direction with its outer circumferential surface 37Ds facing the transport path of the continuous sheet 3a of the top sheet. The servomotor serves as a drive source that drives to rotate the rotating drum 37D. On the outer circumferential surface 37Ds of the rotating drum 37D, a suction force is caused by suction, and thus the plurality of the cushion sheets 8, 8... are held on the outer circumferential surface 37Ds while being arranged side-by-side at the product pitch P1 in the direction-of-rotation Dc37. Due to the rotation of the rotating drum 37D, each cushion sheet 8 is transferred toward a facing position P3a2 (the same position as the abovementioned predetermined position P8 and corresponding to a "third processing position") which faces the continuous sheet 3a. When the cushion sheet 8 passes through the facing position P3a2, the cushion sheet 8 is bonded onto the non-skin-side surface of the continuous sheet 3a with adhesive. Thus, the cushion sheet 8 is transferred from the outer circumferential surface 37Ds of the rotating drum 37D to the continuous sheet 3a.

Note that, as in the abovementioned case of the fastening-tape-member bonding device 34, the rotation operation of the rotating drum 37D is basically operated according to a synchronization signal. That is, the servomotor of the rotating drum 37D is controlled in terms of position so that a position at which the cushion sheet 8 is sucked and held on the outer circumferential surface 37Ds is moved to an instruction position in the direction-of-rotation Dc37 indicated by the synchronization signal. This basically causes the cutform cushion sheets 8, which are arranged at the product pitch P1 on the outer circumferential surface 37Ds, to be transported in the direction-of-rotation Dc37 by the product pitch P1, each time a unit signal of the synchronization signal is outputted.

Meanwhile, in the third embodiment also as in the abovementioned first embodiment, the CD-direction position adjustment device 35 is arranged downstream in the direction of transport of and next to the fastening-tape-member bonding device 34. However, on the downstream side of the CD-direction position adjustment device 35, the abovementioned gathering position Pj is not positioned next to the CD-direction position adjustment device 35, but instead the cushion-sheet bonding device 37 is positioned next to the CD-direction position adjustment device 35. Thus, the CD-direction position adjustment device 35 in the third embodiment is used for a purpose different from the purpose, in the abovementioned first embodiment "for bonding the continuous sheet 3a of the top sheet onto the appropriate position in the CD-direction on the continuous sheet 4a of the back sheet". That is, the CD-direction position adjustment device 35 adjusts, in the CD direction, the positions of the end portions 3ae and 3ae of the continuous sheet 3a, to bond the cushion sheet 8 onto the appropriate position in the CD-direction on the continuous sheet 3a of the top sheet.

Further, in the third embodiment, as described above, the CD-direction position adjustment device 38 (corresponding to a third position adjustment device), which is different from the abovementioned CD-direction position adjustment device 35, is additionally provided downstream in the direction of transport with respect to the cushion-sheet bonding device 37 and at the predetermined position P38 upstream with respect to the gathering position Pj. The CD-direction position adjustment device 38 also adjusts, in the CD direction, the positions of the end portions 3ae and 3ae in the CD-direction of the continuous sheet 3a of the top sheet independently of each other, such that the end portions 3ae and 3ae are positioned at the target positions in the CD direction, respectively. With such adjustment, the continuous sheet 3a of the top sheet is brought into an appropriate relative positional relationship in terms of the CD direction with the continuous sheet 3a of the back sheet and the absorbent body 2 that are to meet the continuous sheet 3a at the gathering position Pj, and then bonded with these continuous sheet 4a and absorbent body 2.

The basic configuration of such a CD-direction position adjustment device 38 is substantially the same as that of the CD-direction position adjustment device 35 which has been already mentioned with reference to Figs. 4 to 5B. That is, although not being shown in detail, the CD-direction position adjustment device 38 in Fig. 12 also includes position adjustment mechanisms 38A and 38A for adjusting the positions of the end portions 3ae and 3ae in the CD-direction of the continuous sheet 3a of the top sheet which are provided on the right and left sides in the CD direction corresponding to the end portions 3ae and 3ae, respectively. These right and left position adjustment mechanisms 38A and 38A are in a mirror image relationship with each other with respect to the center line CL (Fig. 5A) of the CD direction and have substantially the same basic configuration. Each position adjustment mechanism 38A includes: a pair of upper and lower nip rollers 38Ru and 38Rd; an actuator (not shown); a sensor (not shown); and a controller (not shown), for example. The upper and lower nip rollers 38Ru and 38Rd in a pair are arranged and driven to rotate with their outer circumferential surfaces facing each other so as to nip the end portion 3ae in the CD-direction of the continuous sheet 3a from two sides in the thickness direction. The actuator drives to turn the pair of upper and lower nip rollers 38Ru and 38Rd about a support shaft (not shown) in a plane including both the direction of transport and the CD direction. The sensor such as a phototube, and/or the like is arranged immediately downstream of the nip rollers 38Ru and 38Rd to measure the position of the end portion 3ae in the CD-direction and output a measurement signal. The controller controls the abovementioned actuator based on the measurement signal from the sensor. The configurations of the abovementioned elements are substantially the same as those of the abovementioned CD-direction position adjustment device 35. Thus, further descriptions thereof are omitted.

As shown in Fig. 12, in the third embodiment as well, the same detecting device 36 as in the first embodiment is included. The detecting device 36 is used to detect the bonding portion of the fastening-tape member 6 on the continuous sheet 3a of the top sheet, and the tension adjustment device 33 in the top-sheet supply line L3aS adjusts the target value of the tension of the continuous sheet 3a of the top sheet based on the detection result. This makes it possible to reduce the amount of deviation in the direction of transport of the positions of the bonding portions of the fastening-tape members 6 and 6 which may be caused by the operation of the CD-direction position adjustment device 35 that is positioned upstream out of the two CD-direction position adjustment devices 35 and 38, that is, the CD-direction position adjustment device 35 that is positioned downstream of and next to the fastening-tape-member bonding device 34.

On the other hand, in the case where the other CD-direction position adjustment device 38 operates, the position of the bonding portion of the cushion sheet 8 in the direction of transport on the continuous sheet 3a of the top sheet may deviate from the appropriate position for the similar reason as described above. When the continuous sheet 3a is transferred to the gathering position Pj as it is, the continuous sheet 3a is bonded to the continuous sheet 4a of the back sheet and the absorbent body 2 at the gathering position Pj in the state where the position of the bonding portion of the cushion sheet 8 on the continuous sheet 3a deviates from the appropriate position (hereinafter referred to as the target position) in the direction of transport. This may cause deterioration of the dimensional accuracy of the diaper 1.

Thus, the position of the bonding portion of the cushion sheet 8 also needs to be adjusted in the direction of transport. In this case, the adjustment is made not by the abovementioned tension adjustment device 33, but by changing the timing when the cushion-sheet bonding device 37 bonds the cushion sheet 8 onto the continuous sheet 3a of the top sheet. This avoids the adjustment of the position of the bonding portion of the cushion sheet 8 from affecting the positions of the bonding portions of the fastening-tape members 6 and 6 on the continuous sheet 3a.

Hereinafter, such an adjustment method will be described.

The adjustment method is implemented as follows, with the detecting device 39 in Fig. 12 and an appropriate control device (not shown) such as a computer and/or the like cooperating with each other.

First, in this example, the abovementioned detecting device 39 detects the bonding portion (corresponding to a trace of a third processing) of the cushion sheet 8 in the continuous sheet 3a at a predetermined position P39 in the direction of transport (corresponding to a third detecting step). The predetermined position P39 (corresponding to a third detection processing position) is set downstream in the direction of transport with respect to the arrangement position P8 (P3a2) of the cushion-sheet bonding device 37 and the arrangement position P38 (the same position as the abovementioned predetermined position P38 and corresponding to a "third adjustment processing position") of the nip rollers 38Ru and 38Rd of the CD-direction position adjustment device 38. The detection result (corresponding to a third detection result) of the bonding portion is transmitted to the abovementioned control device each time the bonding portion is detected.

Then, each time the detection result is received, the control device changes timing in which the rotating drum 37D of the cushion-sheet bonding device 37 transfers the cushion sheet 8 to the abovementioned facing position P3a2, from the timing indicated by the synchronization signal. Consequently, the amount of deviation of the position of the abovementioned bonding portion on the continuous sheet 3a from the target position is reduced.

For example, in the case where the detection result indicates that the position of the bonding portion of the cushion sheet 8 deviates from the target position on the continuous sheet 3a by a predetermined amount of deviation to a downstream side in the direction of transport, the control device decreases the abovementioned instruction position indicated by the synchronization signal by an adjustment value obtained by multiplying the amount of deviation by a predetermined gain. On the other hand, in the case where the detection result indicates that the position of the bonding portion deviates from the target position by a predetermined amount of deviation to an upstream side in the direction of transport, the control device increases the abovementioned instruction position by an adjustment value obtained by multiplying the amount of deviation by a predetermined gain.

The configuration example of such a detecting device 39 is substantially the same as that of the detecting device 36 described in the abovementioned first embodiment. That is, as shown in Fig. 12, the detecting device 39 includes an imaging device 39C such as a CCD camera and/or the like and a control section 39CN such as a computer and/or the like.

The imaging device 39C generates image data of the continuous sheet 3a by imaging the continuous sheet 3a at the abovementioned predetermined position P39 (hereinafter referred to as an imaging position P39 which is the same position as the abovementioned predetermined position P39). Such an imaging operation is performed each time the rotational angle value of the synchronization signal received by the imaging device 39C matches the defined rotational angle that is prestored in a memory of the imaging device 39C. Further, the imaging operation is performed with such a field of view as to include at least a part of the cushion sheet 8. Each time the image data is transmitted from the imaging device 39C, the control section 39CN analyzes the image data, thereby obtaining information on the amount of deviation of the position of the bonding portion from the target position on the continuous sheet 3a of the top sheet. For example, the position of the bonding portion on the image data is obtained by binarization process and/or the like, and then the amount of deviation of the position of the bonding portion from the target position on image data which is prestored in a memory of the control section 39CN is calculated. This is used as information on the amount of deviation indicated by the abovementioned detection result.

In the third embodiment, as described above, the amount of deviation of the position of the bonding portion formed by the fastening-tape-member bonding device 34 upstream in the direction of transport is reduced by adjusting the tension with the abovementioned tension adjustment device 33, and meanwhile, the amount of deviation of the position of the bonding portion formed by the cushion-sheet bonding device 37 downstream therefrom is reduced by changing the timing of bonding by the cushion-sheet bonding device 37 itself.

Thus, in this example, the processing of reducing the amount of deviation of the position of the bonding portion of the cushion sheet 8 does not affect the bonding processing by the fastening-tape-member bonding device 34 located upstream from the cushion-sheet bonding device 37. In this regard, this example is advantageous.

That is, if such a correspondence relationship is opposite, the relationship is as follows: the amount of deviation of the position of the bond portion formed by the fastening-tape-member bonding device 34 is reduced by changing the timing of bonding by the fastening-tape-member bonding device 34 itself, while the amount of deviation of the position of the bonding portion formed by the cushion-sheet bonding device 37 is reduced by adjusting the tension with the abovementioned tension adjustment device 33 located further upstream in the direction of transport from the fastening-tape-member bonding device 34.

In such a case, however, the adjustment of the tension causes the position of the continuous sheet 3a to change in the direction of transport, also at the position in the direction of transport of the fastening-tape-member bonding device 34. This may results in a great amount of deviation of the position of the bonding portion of the fastening-tape member 6 from the target position. In this regard, such an issue can be avoided in the abovementioned correspondence relationship.

Other Embodiments

While embodiments and the like according to the present disclosure are described, the above embodiments of the present disclosure are for facilitating understanding of the present disclosure, and are not limiting of the present disclosure. The present disclosure can of course be altered and improved without departing from the gist thereof, and equivalents are intended to be embraced therein. For example, the present disclosure can be altered as described below.

In the foregoing embodiments and the like, an absorbent article is exemplified by a so-called tape-type disposable diaper 1. However, it is not limited thereto. For example, a pull-on disposable diaper may be employed. As a pull-on disposable diaper, a so-called three-piece diaper may be employed in which a liquid-permeable absorbent main body (a sheet-like member having the absorbent body 2 therein) stretches between a front band member and a back band member when the diaper is unfolded, or alternatively a so-called two-piece diaper may be employed in which an absorbent main body is placed on the skin-side surface of an exterior sheet having a substantially hour-glass shape when the diaper is unfolded. Furthermore, the absorbent article is not limited to the disposable diaper 1. That is, it may be any article that absorbs excreted fluid from the wearer. For example, the absorbent article may be a sanitary napkin, a urine absorbing pad, and/or the like.

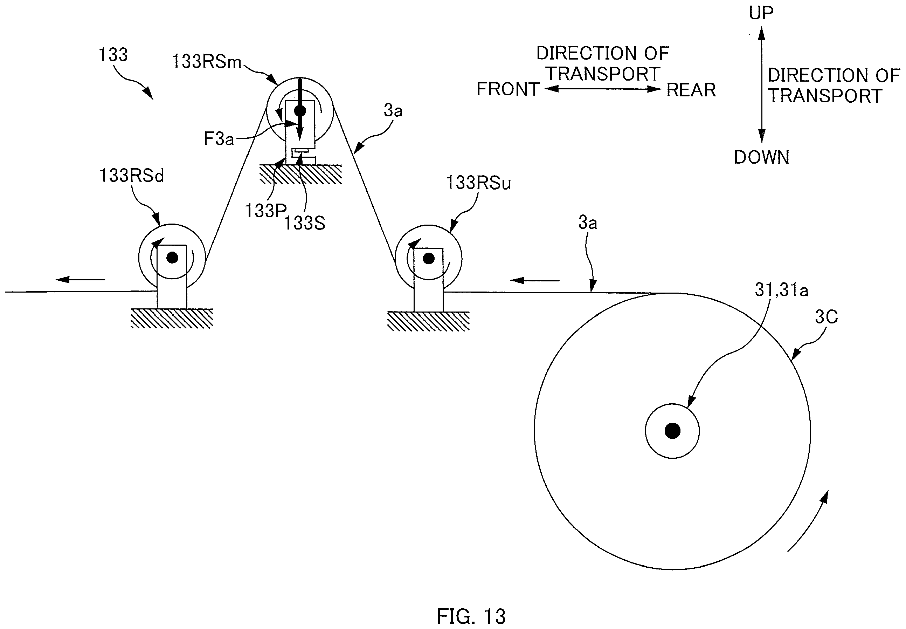

In the foregoing embodiments and the like, as shown in FIG. 4, a dancer-roll type one is provided as an example of the tension adjustment device 33. However, it is not limited thereto. That is, a device may be employed as long as the device measures the tension of the continuous sheet 3a and can change the feeding speed (mpm) of the feeding device 31 so that the tension becomes the target value. FIG. 13 is a diagram illustrating a tension adjustment device 133, which is an example thereof. First, instead of the dancer roll 33RD and the pair of stationary rolls 33RSu and 33RSd located upstream and downstream therefrom shown in FIG. 4, the tension adjustment device 133 of FIG. 13 includes three rolls 133RSu, 133RSm and 133RSd at fixed positions in the direction of transport. The three rolls 133RSu, 133RSm and 133RSd are supported rotatably about the rotation shaft extending along the CD direction. Of the three rolls 133RSu, 133RSm and 133RSd, the center roll 133RSm is supported by a support portion 133P, in which a sensor 133S such as a strain gauge, and/or the like is provided. The abovementioned sensor 133S outputs in real time a value depending on force F3a which is applied by the continuous sheet 3a to the center roll 133RSm while the continuous sheet 3a being run about the three roll 133RSu, 133RSm and 133RSd in a chevron form. In addition, the value outputted by the sensor 133S is converted into a tension in real time by an appropriate converter (not shown). The feeding speed (mpm) of the feeding device 31 may be changed so that the tension (N) becomes the abovementioned target value.

In the foregoing embodiments and the like, the processing which is performed onto portions each corresponding to an absorbent article of the continuous sheet at intervals in the direction of transport is exemplified by bonding of the pair of fastening-tape members 6 and 6 to the continuous sheet 3a of the top sheet, and further the second processing which is performed onto portions each corresponding to the absorbent article of the continuous sheet at intervals in the direction of transport is exemplified by bonding of the leak-proof sheet 5 to the continuous sheet 4a of the back sheet, and bonding of the target tape 7 to the continuous sheet 4a. However, the processing and the second processing are not limited thereto. For example, the processing and the second processing may be applying adhesive onto the corresponding continuous sheets 3a and 4a at intervals in the direction of transport, or may be intermittently-pressing, welding, cutting, or the like.

In the foregoing embodiments and the like, as shown in FIG. 45, the tension adjustment device 33 is exemplified by one including: the stationary rolls 33RSu and 33RSd in a pair arranged at respective fixed positions and provided rotatably about the rotation shaft extending along the CD direction; the dancer roll 33RD guided so as to be movable in the predetermined direction (up-down direction in FIG. 4) at a position between the pair of stationary rolls 33RSu and 33RSd while being rotatable about the rotation shaft extending along the CD direction; the actuator applies load FD to the dancer roll 33RD in a direction of increasing the size of a loop in the predetermined direction, the load FD corresponding to the target value of the tension of the continuous sheet 3a; the measuring device that detects the position of the dancer roll 33RD in the predetermined direction and outputs positional information; and the control section to which the positional information is inputted. The tension adjustment device 33 changes the foregoing target value of the tension based on the detection result of the bonding portion of the fastening-tape members 6, 6 in the continuous sheet 3a of the top sheet, to adjust the tension at the time of transferring the continuous sheet 3a to the top-sheet processing line L3aK. However, it is not limited thereto.

That is, in the case where the stationary roll 33RSd, located downstream in the direction of transport is a driving roll, out of the pair of stationary rolls 33RSu and 33RSd shown in FIG. 4 is driven to be rotated by a servomotor serving as a drive source, the rotation speed (mpm) of the driving roll 33RSd may be changed based on the abovementioned detection result instead of changing the target value of the tension.

For example, in the case where the detection result indicates that the bonding portions of the fastening-tape members 6 and 6 are located downstream in the direction of transport from the target position on the continuous sheet 3a, the rotation speed (mpm) of the driving roll 33RSd is decreased to be smaller than the current value. This increases the tension at the time of transferring the continuous sheet 3a to the top-sheet processing line L3aK. On the contrary, in the case where the detection result indicates that the bonding portion is located upstream in the direction of transport from the target position, the rotation speed (mpm) is increased to be larger than the current value. This decreases the tension at the time of transferring.

This makes it possible to decrease the amount of deviation in the direction of transport of the positions of the bonding portions of the fastening-tape members 6 and 6 from the target positions which can be caused by an adjustment in the CD direction of the end portions 3ae and 3ae in the CD direction in the continuous sheet 3a

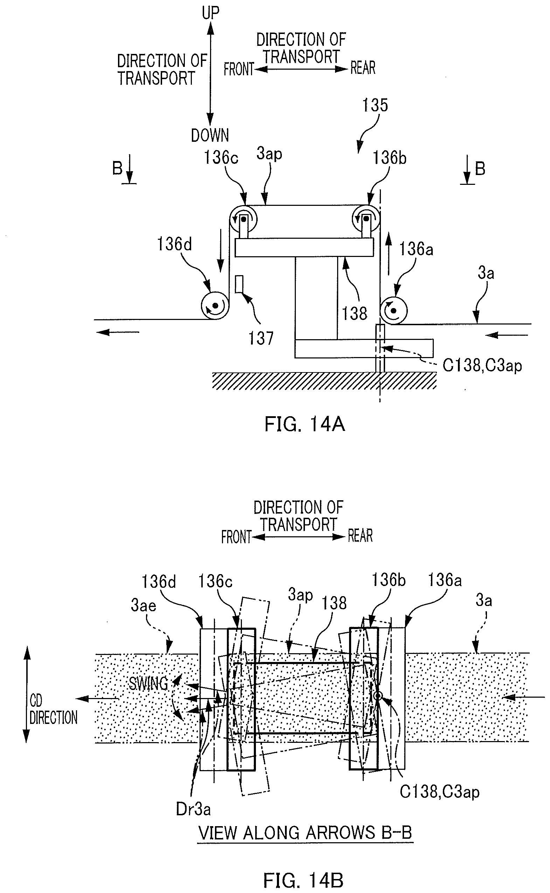

In the abovementioned embodiments and the like, as shown in Fig. 5A, the CD-direction position adjustment device 35 is exemplified by the device 35 that adjusts, in the CD direction, the positions of the end portions 3ae and 3ae in the CD-direction on the continuous sheet 3a independently of each other, however, it is not limited thereto. That is, a device is usable as long as the device adjusts, in the CD direction, the positions of the end portions 3ae in the CD-direction of the continuous sheet 3a as a result. Figs. 14A and 14B are explanatory diagrams of a CD-direction position adjustment device 135 as an example of such a device. Fig. 14A is a schematic side view of the CD-direction position adjustment device 135, and Fig. 14B is a cross-sectional view taken along line B-B of Fig. 14A. In Fig. 14B, the continuous sheet 3a is shown by a dashed triple-dotted line in the transparent state.

As shown in Fig. 12A, the CD-direction position adjustment device 135 (corresponding to a position adjustment device) includes four rollers 136a, 136b, 136c, and 136d and a sensor 137. The four rollers 136a, 136b, 136c, and 136d transport the continuous sheet 3a through the transport path having a substantially square U-shape seen from a side view in the CD direction. The sensor 137, such as a photoelectric tube and/or the like, detects the position in the CD-direction of at least one of the end portions 3ae in the CD-direction of the continuous sheet 3a. The four rollers 136a, 136b, 136c, and 136d are arranged side-by-side in the direction of transport. The roller 136a on the most downstream side and the roller 136d on the most upstream side are provided rotatably about the rotation shafts extending along the CD direction at fixed positions in the direction of transport, respectively. The rest two rollers 136b and 136c are, as shown in Fig. 12B, supported by a predetermined swing rotation member 138 with their rotation shafts maintained in parallel to each other. The swing rotation member 138 is supported so as to be swingably rotatable about a support shaft C138 arranged at a fixed position. The support shaft C138 is parallel to the direction of the normal of a portion 3ap of the continuous sheet 3a stretching between the abovementioned two rollers 136b and 136c. The support shaft C138 is arranged at a center position C3ap in the CD direction of an upstream end of the abovementioned portion 3ap in a state where the continuous sheet 3a is located at the target position in the CD direction. When an appropriate actuator (not shown) such as a feed screw mechanism including a servomotor, and/or the like swing the swing rotation member 138 about the abovementioned support shaft C138, thereby being able to tilt a direction Dr3a of transferring the continuous sheet 3a with the two rollers 136b and 136c to one side or the other side in the CD direction from a direction orthogonal to the CD direction. Consequently, such a swing operation is performed based on the information on the position in the CD-direction of the end portion 3ae outputted from the sensor 137, thereby being able to adjust the position in the CD-direction of the continuous sheet 3a to the target position in the CD direction.

In the abovementioned third embodiment, as shown in Fig. 12, the imaging position P36 of the upstream detecting device 36 out of the two detecting devices 36 and 39 is set at a position between the arrangement position P35 of the nip rollers 35Ru and 35Rd of the upstream CD-direction position adjustment device 35 and the arrangement position P38 of the nip rollers 38Ru and 38Rd of the downstream CD-direction position adjustment device 38 (or the arrangement position P8 of the cushion-sheet bonding device 37); however, it is not limited thereto. For example, the imaging position P36 may be set downstream from the abovementioned arrangement position P38 of the downstream CD-direction position adjustment device 38. As long as the imaging position P36 is set at such a position, the amount of deviation of the position in the direction of transport of the bonding portion of the fastening-tape member 6 which has been caused by the operation of the downstream CD-direction position adjustment device 35 is also reflected on the detection result of the detecting device 36. Accordingly, the tension adjustment device 33 is capable of adjusting the target value of the tension more appropriately so as to reduce the amount of deviation. Regarding the relative positional relationship with the gathering position Pj, the imaging position P36 may be set upstream from the gathering position Pj or may be set downstream from the gathering position Pj.

[Reference Signs List]

1 disposable diaper (absorbent article), 1LH leg opening,
1a substrate of diaper, 1f front part, 1b back part, 1p portion corresponding to a diaper,
2 absorbent body,
3 top sheet,
3a continuous sheet of top sheet (continuous sheet, first continuous sheet),
3ae end portion, 3ap portion, 3C material coil,
4 back sheet,
4a continuous sheet of back sheet (second continuous sheet), 4ae end portion, 4C material coil,
5 leak-proof sheet, 6 fastening-tape member, 6m male member,
7 target tape, 8 cushion sheet,
31 feeding device, 31a rotation-shaft portion,
33 tension adjustment device, 33RD dancer roll, 33RSu stationary roll, 33RSd stationary roll,
33A tension adjustment device (upstream tension adjustment device), 33ARD dancer roll,
33ARSu stationary roll, 33ARSd stationary roll,
33B tension adjustment device (downstream tension adjustment device), 33BRD dancer roll,
33BRSu stationary roll, 33BRSd stationary roll,
34 fastening-tape-member bonding device (processing device, first processing device),
34D rotating drum, 34Ds outer circumferential surface,
35 CD-direction position adjustment mechanism (position adjustment device, first position adjustment device),
35A position adjustment mechanism, 35Ru nip roller, 35Rd nip roller,
35d actuator, 35s sensor,
36 detecting device, 36C imaging device, 36CN control section,
37 cushion-sheet bonding device (third processing device),
37D rotating drum, 37Ds outer circumferential surface,
38 CD-direction position adjustment device (third position adjustment device),
38A position adjustment mechanism, 38Ru nip roller, 38Rd nip roller,
39 detecting device, 39C imaging device, 39CN control section,
41 feeding device, 41a rotation-shaft portion,
43 tension adjustment device, 43RD dancer roll,
43RSu stationary roll, 43RSd stationary roll,
45 leak-proof-sheet bonding device (second processing device),
45D rotating drum, 45Ds outer circumferential surface,
47 target-tape bonding device (second processing device),
47D rotating drum, 47Ds outer circumferential surface,
49 CD-direction position adjustment device (second position adjustment device), 49A position adjustment mechanism,
49Ru nip roller, 49Rd nip roller, 49d actuator,
49s sensor,
111 bonding device, 111u press roll, 111d press roll,
112 leg-opening cutting device, 112u upper roll, 112d lower roll,
116 end-cutting device, 116u upper roll, 116d lower roll,
133 tension adjustment device,
133Rsu roll, 133RSm roll, 133RSd roll,
133P support portion, 133S sensor,
135 CD-direction position adjustment device (position adjustment device),
136a roller, 136b roller, 136c, 136d roller,
137 sensor, 138 swing rotation member,
TB turn bar (transport-direction-changing member),
CV belt conveyer,
L1a substrate processing system, L2 absorbent-body producing system,
L3a top-sheet processing system, L3a' top-sheet processing system,
L3a" top-sheet processing system,
L3aK top-sheet processing line (processing section)
L3aK' top-sheet processing line (processing section)
L3aK" top-sheet processing line (processing section)
L3aS top-sheet supply line,
L4a back-sheet processing system, L4aK back-sheet processing line (second processing section),
L4aS back-sheet supply line (supplying section), LM manufacturing line,
C3ap center position, CL center line, C35A support shaft, C49A support shaft, C138 support shaft,
Pj gathering position, P1ac position,
P3a facing position (processing position, first processing position),
P3a2 facing position (third processing position),
P4a1 facing position (second processing position),
P4a2 facing position (second processing position),
P5 predetermined position (second processing position),
P6 predetermined position (processing position, first processing position),
P7 predetermined position (second processing position), P8 predetermined position (third processing position),
P35 arrangement position (predetermined position, adjustment processing position, first adjustment processing position),

P36 imaging position (detection processing position),

P38 arrangement position (third adjustment processing position),

P39 imaging position (third detection processing position),

P49 predetermined position, P112 predetermined position, P116 position

**Claims**

1. A method for manufacturing a sheet-like member associated with an absorbent article (1) by using a continuous sheet (3a), the method comprising:

a supplying step of transferring and supplying the continuous sheet (3a) to a processing section (L3aK) by transporting the continuous sheet (3a) in a direction of transport that conforms to a continuous direction in which the continuous sheet (3a)continues;

a processing step of performing processing onto portions of the continuous sheet (3a) at intervals in the direction of transport, at a processing position (P3a) in the direction of transport, using a processing device (34) in the processing section (L3aK), the portions each corresponding to the absorbent article (1);

a position adjusting step of adjusting, in a CD direction, a position of an end portion (3ae) in the CD direction in the continuous sheet (3a), the CD direction intersecting the direction of transport in the continuous sheet (3a), the position adjusting step being performed, by a position adjustment device (35), at an adjustment processing position (P35) in the direction of transport; and

a detecting step of detecting a trace of the processing left on the continuous sheet (3a), at a detection processing position (P36) downstream in the direction of transport from the processing position (P3a) and the adjustment processing position (P35), and outputting a detection result,

the supplying step including adjusting, based on the detection result, a tension in the direction of transport of the continuous sheet (3a) at a time of being transferred to the processing section (L3aK), at a position upstream in the direction of transport from the processing position (P3a),

wherein the adjustment processing position (P35) is located downstream in the direction of transport from the processing position (P3a).

2. The method for manufacturing a sheet-like member associated with an absorbent article according to claim 1, wherein

when the detection result indicates that the trace of the processing is located downstream in the direction of transport from a target position on the continuous sheet (3a), the tension is increased, and

when the detection result indicates that the trace of the processing is located upstream in the direction of transport from the target position, the tension is decreased.

3. The method for manufacturing a sheet-like member associated with an absorbent article according to claim 1, wherein

when the continuous sheet (3a) is defined as a first continuous sheet,

a second continuous sheet different from the first continuous sheet is transported along a continuous direction in which the second continuous sheet continues, the continuous direction conforming to the direction of transport, while a bonding device layers and bonds the first continuous sheet and the second continuous sheet together at a gathering position (Pj) downstream in the direction of transport from the adjustment processing position (P35), and

the detection processing position (P36) is located downstream in the direction of transport from the gathering position (Pj).

4. An apparatus for manufacturing a sheet-like member associated with an absorbent article (1) by using a continuous sheet (3a), the apparatus comprising:

a supplying section that transfers and supplies the continuous sheet (3a) to a processing section (L3aK) by transporting the continuous sheet (3a) in a direction of transport that conforms to a continuous direction in which the continuous sheet (3a) continues;

a processing device (34) that performs processing onto portions of the continuous sheet (3a) at intervals in the direction of transport, at a processing position (P3a) in the direction of transport in the processing section (L3aK), the portions each corresponding to the absorbent article (1);

a position adjustment device (35) that adjusts, in a CD direction, a position of an end portion in the CD direction of the continuous sheet (3a), at an adjustment processing position (P35) in the direction of transport, the CD direction intersecting the direction of transport in the continuous sheet (3a); and

a detecting device that detects a trace of the processing left on the continuous sheet, at a detection processing position (P36) downstream in the direction of transport from the processing position and the adjustment processing position (P35), and outputs a detection result,

the supplying section being configured to adjust, based on the detection result, a tension in the direction of transport of the continuous sheet (3a) at a time of being transferred to the processing section, at a position upstream in the direction of transport from the processing position (P3a),

wherein the adjustment processing position (P35) is located downstream in the direction of transport from the processing position (P3a).

**Patentansprüche**

1. Verfahren zur Herstellung eines bahnförmigen Elements, das mit einem saugfähigen Artikel (1) verknüpft ist, unter Verwendung einer durchgehenden Bahn (3a), wobei das Verfahren Folgendes umfasst:

   einen Zuführungsschritt, der darin besteht, die durchgehende Bahn (3a) zu überführen und einem Verarbeitungsbereich (L3aK) zuzuführen, indem die durchgehende Bahn (3a) in einer Transportrichtung transportiert wird, die mit einer durchgehenden Richtung übereinstimmt, in welcher die durchgehende Bahn (3a) weitergeht;
   einen Verarbeitungsschritt, der darin besteht, eine Verarbeitung an Abschnitten der durchgehenden Bahn (3a) in Intervallen in der Transportrichtung in einer Verarbeitungsposition (P3a) in der Transportrichtung unter Verwendung einer Verarbeitungsvorrichtung (34) in dem Verarbeitungsbereich (L3aK) auszuführen, wobei die Abschnitte jeweils dem saugfähigen Artikel (1) entsprechen;
   einen Positionsanpassungsschritt, der darin besteht, in einer CD-Richtung eine Position eines Endabschnitts (3ae) in der CD-Richtung in der durchgehenden Bahn (3a) anzupassen, wobei sich die CD-Richtung mit der Transportrichtung in der durchgehenden Bahn (3a) schneidet, wobei der Positionsanpassungsschritt von einer Positionsanpassungsvorrichtung (35) in einer Anpassungsverarbeitungsposition (P35) in der Transportrichtung ausgeführt wird; und
   einen Detektionsschritt, der darin besteht, Rückstände der Verarbeitung, die auf der durchgehenden Bahn (3a) zurückbleiben, in einer Detektionsverarbeitungsposition (P36) stromabwärts in der Transportrichtung im Verhältnis zu der Verarbeitungsposition (P3a) und der Anpassungsverarbeitungsposition (P35) zu detektieren und ein Detektionsergebnis auszugeben, wobei der Zuführungsschritt basierend auf dem Detektionsergebnis das Anpassen einer Spannung in der Transportrichtung der durchgehenden Bahn (3a) zu einem Zeitpunkt, zu dem sie auf den Verarbeitungsbereich (L3aK) überführt wird, in einer Position stromaufwärts in der Transportrichtung im Verhältnis zu der Verarbeitungsposition (P3a) umfasst, wobei sich die Anpassungsverarbeitungsposition (P35) stromabwärts in der Transportrichtung im Verhältnis zu der Verarbeitungsposition (P3a) befindet.

2. Verfahren zur Herstellung eines bahnförmigen Elements, das mit einem saugfähigen Artikel verknüpft ist, nach Anspruch 1, wobei, wenn das Detektionsergebnis angibt, dass sich die Rückstände der Verarbeitung in der Transportrichtung im Verhältnis zu einer Zielposition stromabwärts auf der durchgehenden Bahn (3a) befinden, die Spannung erhöht wird, und wenn das Detektionsergebnis angibt, dass sich die Rückstände der Verarbeitung in der Transportrichtung im Verhältnis zu der Zielposition stromaufwärts befinden, die Spannung verringert wird.

3. Verfahren zur Herstellung eines bahnförmigen Elements, das mit einem saugfähigen Artikel verknüpft ist, nach Anspruch 1, wobei, wenn die durchgehende Bahn (3a) als eine erste durchgehende Bahn definiert ist, eine zweite durchgehende Bahn, die anders als die erste durchgehende Bahn ist, entlang einer durchgehenden Richtung transportiert wird, in welcher die zweite durchgehende Bahn weitergeht, wobei die durchgehende Richtung mit der Transportrichtung übereinstimmt, während eine Klebevorrichtung die erste durchgehende Bahn und die zweite durchgehende Bahn in einer Sammelposition (Pj) stromabwärts in der Transportrichtung im Verhältnis zu der Anpassungsverarbeitungsposition (P35) zusammen schichtet und verklebt, und sich die Detektionsverarbeitungsposition (P36) stromabwärts in der Transportrichtung im Verhältnis zu der Sammelposition (Pj) befindet.

4. Gerät zur Herstellung eines bahnförmigen Elements, das mit einem saugfähigen Artikel (1) verknüpft ist, unter Verwendung einer durchgehenden Bahn (3a), wobei das Gerät Folgendes umfasst:

   einen Zuführungsbereich, der die durchgehende Bahn (3a) überführt und einem Verarbeitungsbereich (L3aK) zuführt, indem die durchgehende Bahn (3a) in einer Transportrichtung transportiert wird, die mit einer durchgehenden Richtung übereinstimmt, in welcher die durchgehende Bahn (3a) weitergeht;
   eine Verarbeitungsvorrichtung (34), die eine Verarbeitung an Abschnitten der durchgehenden Bahn (3a) in Intervallen in der Transportrichtung in einer Verarbeitungsposition (P3a) in der Transportrichtung in dem Verarbeitungsbereich (L3aK) ausführt, wobei die Abschnitte jeweils dem saugfähigen Artikel (1) entsprechen; eine Positionsanpassungsvorrichtung (35), die in einer CD-Richtung eine Position eines Endabschnitts in der CD-Richtung der durchgehenden Bahn (3a) in einer Anpassungsverarbeitungsposition (P35) in der Transportrichtung anpasst, wobei sich die CD-Richtung mit der Transportrichtung in der durchgehenden Bahn (3a) schneidet; und eine Detektionsvorrichtung, die Rückstände der Verarbeitung, die auf der durchgehenden Bahn zurückbleiben, in einer Detektionsverarbeitungsposition (P36) stromabwärts in der Transportrichtung im Verhältnis zu der Verarbeitungsposition und der Anpassungsverarbeitungsposition (P35) detektiert und ein Detektionsergebnis ausgibt, wobei der Zuführungsbereich konfiguriert ist, um basierend auf dem Detektionsergebnis eine Spannung in der Transportrichtung der durchgehenden Bahn (3a) zu einem Zeitpunkt, zu dem sie auf den Verarbeitungsbereich überführt wird, in einer Position stromaufwärts in der Transportrichtung im Verhältnis zur Verarbeitungsposition (P3a) anzupassen, wobei sich die Anpassungsverarbeitungsposition (P35) in der Transportrichtung im Verhältnis zu der Verarbeitungsposition (P3a) stromabwärts befindet.

## Revendications

1. Procédé de fabrication d'un élément de type feuille associé à un article absorbant (1) en utilisant une feuille continue (3a), le procédé comprenant :

   une étape de fourniture consistant à transférer et à fournir la feuille continue (3a) à une section de traitement (L3aK) en transportant la feuille continue (3a) dans une direction de transport qui se conforme à une direction continue dans laquelle la feuille continue (3a) continue ; une étape de traitement consistant à réaliser un traitement sur des portions de la feuille continue (3a) à des intervalles dans la direction de transport, à une position de traitement (P3a) dans la direction de transport, à l'aide d'un dispositif de traitement (34) dans la section de traitement (L3aK), les portions correspondant chacune à l'article absorbant (1) ; une étape d'ajustement de position consistant à ajuster, dans une direction CD, une position d'une portion d'extrémité (3ae) dans la direction CD dans la feuille continue (3a), la direction CD coupant la direction de transport dans la feuille continue (3a), l'étape d'ajustement de position étant réalisée, par un dispositif d'ajustement de position (35), à une position de traitement d'ajustement (P35) dans la direction de transport ; et une étape de détection consistant à détecter une trace du traitement laissée sur la feuille continue (3a), à une position de traitement de détection (P36) en aval dans la direction de transport depuis la position de traitement (P3a) et la position de traitement d'ajustement (P35), et à fournir en sortie un résultat de détection, l'étape de fourniture comportant l'ajustement, d'après le résultat de détection, d'une tension dans la direction de transport de la feuille continue (3a) à un moment où elle est transférée à la section de traitement (L3aK), à une position en amont dans la direction de transport depuis la position de traitement (P3a), dans lequel la position de traitement d'ajustement (P35) est située en aval dans la direction de transport depuis la position de traitement (P3a).

2. Procédé de fabrication d'un élément de type feuille associé à un article absorbant selon la revendication 1, dans lequel lorsque le résultat de détection indique que la trace du traitement est située en aval dans la direction de transport depuis une position cible sur la feuille continue (3a), la tension est augmentée, et lorsque le résultat de détection indique que la trace du traitement est située en amont dans la direction de transport depuis la position cible, la tension est diminuée.

3. Procédé de fabrication d'un élément de type feuille associé à un article absorbant selon la revendication 1, dans lequel lorsque la feuille continue (3a) est définie en tant que première feuille continue, une seconde feuille continue différente de la première feuille continue est transportée suivant une direction continue dans laquelle la seconde feuille continue, la direction continue se conformant à la direction de transport, tandis qu'un dispositif de liaison dispose en couches et lie la première feuille continue et la seconde feuille continue ensemble à une position d'assemblage (Pj) en aval dans la direction de transport depuis la position de traitement d'ajustement (P35), et la position de traitement de détection (P36) est située en aval dans la direction de transport depuis la position d'assemblage (Pj).

4. Appareil de fabrication d'un élément de type feuille associé à un article absorbant (1) en utilisant une feuille continue (3a), l'appareil comprenant :

une section de fourniture qui transfère et fournit la feuille continue (3a) à une section de traitement (L3aK) en transportant la feuille continue (3a) dans une direction de transport qui se conforme à une direction continue dans laquelle la feuille continue (3a) continue ;

un dispositif de traitement (34) qui réalise un traitement sur des portions de la feuille continue (3a) à des intervalles dans la direction de transport, à une position de traitement (P3a) dans la direction de transport dans la section de traitement (L3aK), les portions correspondant chacune à l'article absorbant (1) ;

un dispositif d'ajustement de position (35) qui ajuste, dans une direction CD, une position d'une portion d'extrémité dans la direction CD de la feuille continue (3a), à une position de traitement d'ajustement (P35) dans la direction de transport, la direction CD coupant la direction de transport dans la feuille continue (3a) ; et

un dispositif de détection qui détecte une trace du traitement laissée sur la feuille continue, à une position de traitement de détection (P36) en aval dans la direction de transport depuis la position de traitement et la position de traitement d'ajustement (P35), et fournit en sortie un résultat de détection,

la section de fourniture étant configurée pour ajuster, d'après le résultat de détection, une tension dans la direction de transport de la feuille continue (3a) à un moment où elle est transférée à la section de traitement, à une position en amont dans la direction de transport depuis la position de traitement (P3a),

dans lequel la position de traitement d'ajustement (P35) est située en aval dans la direction de transport depuis la position de traitement (P3a).

WIDTH DIRECTION

FRONT

LONGITUDINAL
DIRECTION

BACK

FIG. 1A

WIDTH DIRECTION

SKIN SIDE

THICKNESS
DIRECTION

NON-
SKIN SIDE

B-B CROSS SECTION

FIG. 1B

FIG. 2

FIG. 3

FIG. 4

FIG. 5A

FIG. 5B

VIEW ALONG ARROWS B-B

FIG. 6

FIG. 7

DIRECTION OF
TRANSPORT
DOWNSTREAM ◄──────► UPSTREAM
(FRONT)                    (REAR)

FIG. 8A

CD
DIRECTION
LEFT ◄──────► RIGHT

UP

DOWN

VIEW ALONG ARROWS B–B

FIG. 8B

EP 3 473 219 B1

FIG. 9A

FIG. 9B

FIG. 10A

FIG. 10B

EP 3 473 219 B1

FIG. 11

EP 3 473 219 B1

FIG. 12

FIG. 13

EP 3 473 219 B1

FIG. 14A

VIEW ALONG ARROWS B-B

FIG. 14B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004262556 A
- JP 2008143699 A

- EP 1983395 A1